(19) Europäisches Patentamt / European Patent Office / Office européen des brevets

(11) **EP 3 383 442 B1**

(12) **EUROPEAN PATENT SPECIFICATION**

(45) Date of publication and mention
of the grant of the patent:
**21.06.2023 Bulletin 2023/25**

(21) Application number: **16784466.1**

(22) Date of filing: **17.10.2016**

(51) International Patent Classification (IPC):
*A61L 2/232* (2006.01)     *A47J 31/44* (2006.01)
*A01N 25/10* (2006.01)     *A01N 59/16* (2006.01)
*A61L 2/238* (2006.01)     *C02F 1/72* (2023.01)
*C09D 5/16* (2006.01)      *D06F 35/00* (2006.01)
*F24C 15/00* (2006.01)     *F24C 15/20* (2006.01)
*H05B 6/64* (2006.01)      *A47L 15/42* (2006.01)
*A61L 2/235* (2006.01)     *A61L 9/18* (2006.01)
*C08K 5/00* (2006.01)      *C08K 5/56* (2006.01)
*C02F 103/00* (2006.01)

(52) Cooperative Patent Classification (CPC):
(C-Sets available)
**A61L 2/232; A01N 55/02; A01N 59/16;
A47J 31/4403; A61L 2/238; C02F 1/725;
C09D 5/14; C09D 7/63; D06F 35/008;
F24C 15/005; H05B 6/6402;** A47L 15/42;
A61L 2/235; A61L 9/18; A61L 2202/11;     (Cont.)

(86) International application number:
**PCT/EP2016/074838**

(87) International publication number:
**WO 2017/092920 (08.06.2017 Gazette 2017/23)**

(54) **USE OF (HETERO)POLYOXOMETALATES FOR SIMULTANEOUSLY IMPARTING ANTIMICROBIAL PROPERTIES TO, AND REDUCING THE GROWTH OF A BIOFILM ON A SURFACE OF A SUBSTRATE IN OR ON A HOME APPLIANCE**

VERWENDUNG VON (HETERO)POLYOXOMETALLATEN ZUR GLEICHZEITIGEN ÜBERTRAGUNG ANTIMIKROBIELLER EIGENSCHAFTEN UND ZUR VERRINGERUNG DES WACHSTUMS EINES BIOFILMS AUF EINER OBERFLÄCHE EINES SUBSTRATS IN ODER AUF EINEM HAUSHALTSGERÄT

UTILISATION D'(HÉTÉRO)POLYOXOMÉTALATES PERMETTANT SIMULTANÉMENT DE CONFÉRER DES PROPRIÉTÉS ANTIMICROBIENNES ET DE RÉDUIRE LA CROISSANCE D'UN BIOFILM SUR UNE SURFACE D'UN SUBSTRAT DANS OU SUR UN APPAREIL DOMESTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2015 EP 15197889**

(43) Date of publication of application:
**10.10.2018 Bulletin 2018/41**

(73) Proprietor: **BSH Hausgeräte GmbH
81739 München (DE)**

(72) Inventors:
• **ARTAL LAHOZ, Maria Carmen
50007 Zaragoza (ES)**
• **BISCHOF, Andreas
10407 Berlin (DE)**
• **HANAU, Andreas
12359 Berlin (DE)**
• **LAZARO VILLARROYA, Guillermo Alberto
50005 Zaragoza (ES)**
• **SANZ NAVAL, Javier
50193 Zaragoza (ES)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
**EP-A1- 1 141 210          WO-A1-2014/122225
WO-A1-2014/154432**

(52) Cooperative Patent Classification (CPC): (Cont.)
A61L 2202/23; A61L 2209/21; C02F 2103/002;
C02F 2303/20; C08K 5/0058; C08K 5/56;
D06F 37/26

C-Sets
**A01N 55/02, A01N 25/10;
A01N 59/16, A01N 25/10**

**Description**

The present invention relates to the use of at least one (hetero)polyoxometalate for simultaneously imparting antimicrobial properties to a surface of a substrate in or on a home appliance and reducing the growth of a biofilm on the surface of the substrate in or on a home appliance.

Home appliances get in contact with dust, dirt, food, humidity or human skin. This can lead to hygienic problems in the long run, because microorganisms can be adsorbed on the surface and multiply. This is unaesthetic for the user, it can produce bad odors and in worst case it can lead to health problems if the appliance is not cleaned regularly and sufficiently.

In home appliances, various solutions are applied in order to achieve or maintain an hygienic status. For instance, materials with antimicrobial properties such as silver-ions (e.g. CN 201389539 Y) or polyoxometalates (e.g. WO 2014/154432 A1) are added to the surface material of the home appliance in order to maintain a germ free status. Various studies have shown that the use of silver-ions is not sufficient to keep a surface in a home appliance germ free over a longer period of time. The polyoxometalates offer a wide range of properties and thus have to be selected carefully and specifically for the various application in home appliances.

The use of polyoxometalates is known in the art for several purposes, e.g. in the general area of analytical chemistry (e.g. elemental analysis, electron staining), the use as catalysts including photo catalysts, in biochemistry for inhibiting electron transfer processes and as electron-dense and rigid components in the crystallization of biomolecules (e.g. ribosomes, leading to the 2009 Nobel Prize), and in medicine due to their antiviral and antitumor activity. The use of polyoxometalates as acid and oxidation catalysts is known in industry (e.g. for the hydration of olefins). Furthermore, (hetero)polyoxometalates are used as delignification agents in bleaching.

EP 1 141 210 B1 discloses a method for bleaching laundry or household surfaces comprising:

(i) providing a wash medium with bleaching composition comprising polyoxometalates; and

(ii) contacting a stained substrate for a time and in an amount sufficient to remove the stains, wherein air is employed as a primary source of oxygen atoms for bleaching.

EP 2 765 136 A1 and WO 2014/122225 A1 disclose the use of heteropolyoxometalates for antimicrobial and disinfecting purposes in substrates, paints and coatings. The (hetero)polyoxometalates described therein consist of a (hetero)polyoxometalate anion and a cation, which is selected from quaternary ammonium cations, quaternary phosphonium cations and tertiary sulfonium cations. The cation makes the heteropolyoxometalate more stable, in particular with respect to its thermal stability.

However, while the heteropolyoxometalates described in EP 2 765 136 A1 and WO 2014/122225 A1 show antimicrobial activity, these heteropolyoxometalates are not necessarily useful in the long term reduction of the biofilm growth on a substrate surface.

Accordingly, there is still a need for (hetero)polyoxometalates that are useful in providing antimicrobial properties to the surface of a substrate in or on a home appliance as well as in the long term reduction, preferably in the long term inhibition, of biofilm growth on the surface of a substrate in or on a home appliance. The aim of this invention is therefore to provide an antibacterial surface material within a home appliance in order to reduce the formulation of biofilm and the bacterial growth on the surface of a substrate used in or on a home appliance.

It has now been found that certain (hetero)polyoxometalates are surprisingly useful for both, i.e. for simultaneously imparting antimicrobial properties to a surface of a substrate in or on home appliance and reducing, preferably inhibiting, on a long term time scale the growth of a biofilm on the surface of the substrate in or on a home appliance. A long term time scale means in the present context at least 3 years.

The large number of structural types in (hetero)polyoxometalate chemistry can be broadly split into three classes: heteropolyanions, isopolyanions and Mo-blue and Mo-brown reduced (hetero)polyoxometalate clusters. From this range of possibilities, (hetero)polyoxometalate anions have been extensively researched. Much of this research has examined their catalytic properties with great emphasis on the Keggin $\{XM_{12}O_{40}\}$ and the Wells-Dawson $\{X_2M_{18}O_{62}\}$ (where M = W or Mo and X = a heteroatom) anions which represent the archetypal systems for (hetero)polyoxometalate anions. In particular W-based (hetero)polyoxometalates have shown a good catalytic activity when used as an antimicrobial agent. This is mostly due to the ability of the W-peroxo ligand combination to produce reactive oxygen species (ROS) following a catalytic mechanism.

Besides the fact that the catalytic activity is mainly associated with the transition metal, modifications of the whole molecule can induce improvements in the generation of reactive oxygen species (ROS). Moreover, these modifications can provide the catalyst with chemical or physical properties on demand depending on the final application without compromising the final performance as catalyst. Thus, (hetero)polyoxometalates represent a mature-enough family of promising compounds. Their versatility is a result from their many structures, the ability to delocalize electrons over the surface of the clusters, and the ability to incorporate heteroanions, electrophiles, and ligands. For example, on the one hand, concerning formulation of (hetero)polyanions, heteroatoms such as P, S, Si, Al, Ge, themselves sharing neighboring oxygen atoms could form part of heteroanions such as $SO_4^{2-}$ and $PO_4^{3-}$ that modify the electronic configuration of the (hetero)polyoxometalate. On the other hand, concerning the 3D structure, the framework of transition metal oxyanions may be modified by using different heteroatoms providing an arguably unrivaled structural diversity of molecules that cover the range from 6 to 368 metal ions in a single molecule and are assembled under "one-pot" reaction conditions. At the extreme, these cluster molecules are truly macromolecular and are formed by self-assembly processes.

With all these possibilities to modulate the catalyst in hands, some (hetero)polyoxometalates have shown to have desirable characteristics useful for simultaneously imparting antimicrobial properties to a surface of a substrate in or on a home appliance and reducing the growth of a biofilm on the surface of the substrate in or on a home appliance. These specific (hetero)polyoxometalates are described in the following.

The present invention therefore relates to the use of at least one (hetero)polyoxometalate for simultaneously imparting antimicrobial properties to a surface of a substrate in or on a home appliance and reducing the growth of a biofilm on the surface of the substrate for at least 3 years, wherein the at least one (hetero)polyoxometalate is of the formula (I), (II), (III), (IV) and/or (V):

$$[A_n]^{m+}[XM_qZ_{r-q-o}Z'_oO_s]^{m-} \qquad (I),$$

wherein

m- is the negative charge of the (hetero)polyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
r = 11 or 12,
o = 0 or 1,
s = 37, 38, 39 or 40,
when r = 11, q = 0, 1, 2, 3, 4, 5, 6, 7, 8 or 9,
when r = 12, q = 0, 1, 2, 3, 4, 5, 6, 7, 8, 9 or 10, and
when r = 12, s = 40, X = P and q is 0, 1, 2 or 3, M is not V;

$$[A_n]^{m+}[XM_qZ_{t-q-o}Z'_oO_{24}]^{m-} \qquad (II),$$

wherein
m- is the negative charge of the (hetero)polyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
t = 4 or 6,
o = 0 or 1,
when t = 6, q = 0, 1, 2, 3 or 4,
when t = 4, q = 0, 1 or 2, and
when t = 4 and X = P, q is 1, 2 or 3;

$$[A_n]^{m+}[X_pM_qZ_{6-q-p-o}Z'_oO_{19}]^{m-} \qquad (III),$$

wherein
m- is the negative charge of the (hetero)polyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,

M is selected from Ti, V, Mn, Fe, Co, Ni, Zn and Cr,
o = 0 or 1,
p = 0 or 1, and
q = 0, 1, 2 or 3
when p = 0, M is not V;

$$[A_n]^{m+}[X_2M_qZ_{18-q-o}Z'_oO_{62}]^{m-} \qquad (IV),$$

wherein
m- is the negative charge of the (hetero)polyoxometalate anion,
m+ is the positive charge of the cation(s) A,
Im-I = Im+I
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13, 14, 15 or 16, and
when X = P, q is 1, 2 or 3;

$$[A_n]^{m+}[X_5M_qZ_{30-q-o}Z'_oO_{110}]^{m-} \qquad (V),$$

wherein
m- is the negative charge of the (hetero)polyoxometalate anion,
m+ is the positive charge of the cation(s) A,
Im-I = Im+I
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13 ,14, 15, 16, 17, 18, 19, 20, 21, 22, 23, 24, 25, 26 or 27;

wherein A is selected from one or more cations and comprises at least one cation selected from the group consisting of quaternary ammonium cations, quaternary phosphonium cations and tertiary sulfonium cations, with the proviso that A is not a quaternary ammonium cation when $[XM_qZ_{r-q-o}Z'_oO_s]^m$ is $[SiV_3W_9O_{40}]^7$.

The substrate according to the present invention is a polymer body or a polymer coating.

The present invention furthermore relates to the use of a substrate in or on a home appliance comprising at least one (hetero)polyoxometalate as defined herein, wherein the substrate is a polymer body or a polymer coating.

Moreover, the present invention relates to a home appliance, wherein a substrate as defined herein is used in or on said home appliance, and wherein said substrate comprises at least one (hetero)polyoxometalate as defined herein.

Preferred embodiments of the present invention are defined in the dependent claims.

Description of the drawings

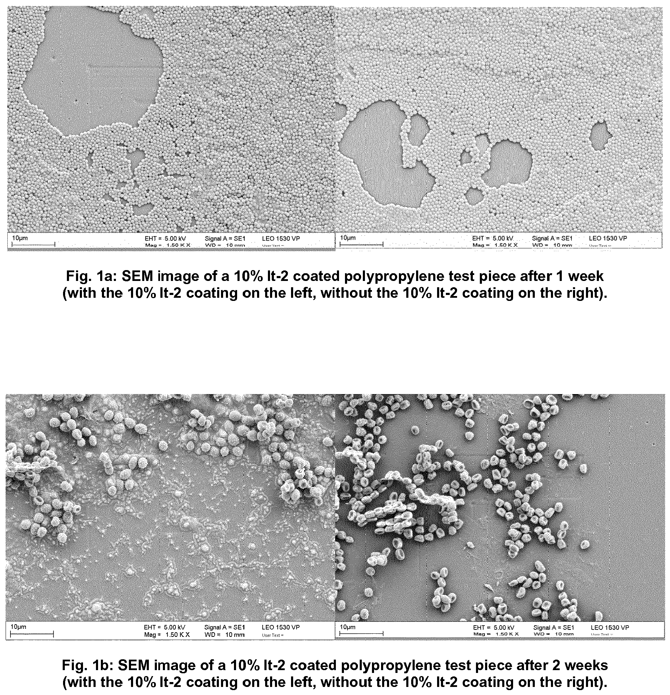

Fig. 1    shows the Staphylococcus aureus biofilm development on a polypropylene test piece coated on one part with an active coating comprising 10% of the heteropolyoxometalate $[(C_4H_9)_3P(C_{14}H_{29})]_7[SiV_3W_9O_{40}]$ (It-2), compared to the uncoated part of the polypropylene piece after 1 week (Fig. 1a) and after 2 weeks (Fig. 1b), as determined by SEM spectroscopy.

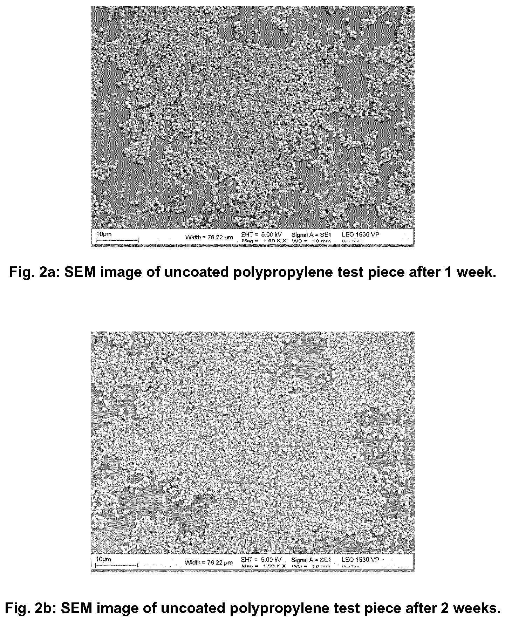

Fig. 2    shows the Staphylococcus aureus biofilm development on a polypropylene piece without (hetero)polyoxometalate-functionalized active coating (blind sample) after 1 week (Fig. 2a) and after 2 weeks (Fig. 2b), as determined by SEM spectroscopy.

Detailed description

The (hetero)polyoxometalates described herein surprisingly show antimicrobial properties and simultaneously reduce the growth of a biofilm on the surface of a substrate in or on a home appliance. The substrate used in a home appliance referred to herein is a polymer body or a polymer coating, wherein the (hetero)polyoxometalate as described herein is incorporated. The antimicrobial (hetero)polyoxometalates described herein are particularly useful in the reduction, preferably in the inhibition, of the growth of a bacteria biofilm on the surface of a substrate in or on a home appliance.

The (hetero)polyoxometalates as described herein consist of a cationic moiety $[A_n]^{m+}$, which imparts antimicrobial activity to the compound, and the (hetero)polyoxometalate anion. The (hetero)polyoxometalates of the present invention show a flexible redox behavior, which means that they can be reversibly reduced by one or more electrons. In particular, it has been found that these (hetero)polyoxometalates, when incorporated into a substrate used in a home appliance, have the ability to activate molecular oxygen and/or hydrogen peroxide, preferably molecular oxygen, which means that an electron is transferred from the (hetero)polyoxometalate to molecular oxygen and/or hydrogen peroxide, preferably molecular oxygen, resulting in the formation of reactive oxygen species (ROS) such as the hyperoxide anion $(O_2^-)$ and an oxidized (hetero)polyoxometalate species. The reactive oxygen species formed by (hetero)polyoxometalate-induced activation of molecular oxygen and/or hydrogen peroxide, preferably of molecular oxygen, reduces the growth of a biofilm on a surface of a substrate in or on a home appliance comprising the (hetero)polyoxometalate. This, in turn, is useful for maintaining the microbial activity of the substrate used in a home appliance resulting from the at least one (hetero)polyoxometalate comprised therein on a long term time scale. A long term time scale, as used in the context of the present invention, means at least 3 years.

The cationic moiety of the oxidized (hetero)polyoxometalate species then reacts with the negatively charged cell membrane of a microorganism thereby killing the microorganism, resulting in an antimicrobial activity of the (hetero)polyoxometalate and the substrate used in a home appliance comprising the (hetero)polyoxometalate, respectively.

The (hetero)polyoxometalates described herein thus provide a synergistic effect: They have antimicrobial properties because of their ability to react with the cell membranes of microorganisms, and they have the ability to generate reactive oxygen species (ROS), in particular the hyperoxide anion $(O_2^-)$, that reduces the growth of a biofilm on the surface of the substrate in or on a home appliance comprising the (hetero)polyoxometalate, with the result that the antimicrobial activity of the substrate used in a home appliance is maintained on a long term time scale ( at least 3 years).

The (hetero)polyoxometalates described herein thus represent heterogeneous catalysts which, because of their flexible redox behavior, regenerate themselves by the reaction with molecular oxygen and/or hydrogen peroxide, preferably molecular oxygen, thereby allowing a long term efficacy of the substrate used in a home appliance comprising the (hetero)polyoxometalate against microorganisms for at least 3 years.

The (hetero)polyoxometalates described herein can also be used for providing self-cleaning, stripping, disinfecting, self-sanitizing, biocidal, antimicrobial, and/or deodorizing properties to at least part of a substrate used in a home appliance or a surface of a substrate in or on a home appliance, and/or for the decomposition and/or degradation of organic materials on a surface of a substrate in or on a home appliance or in the vicinity thereof.

The term "home appliance" as used herein, refers to devices which are intended to be used in private household applications, also including communication- and data processing devices, which are suitable und intended to interact with the devices for private household applications. In particular, the following household applications are included: stoves, ovens, baking ovens, microwaves, hobs, cooker hoods, dishwashers, laundry machines, dryers, refrigerators, freezers, vacuum cleaners, coffee machines, water boilers, plant oil cookers, irons, hair dryers, shavers, kitchen machines and kitchen devices, barbeque devices, steam cookers, electrical small instantaneous water heaters, devices for heating and storing water for the kitchen and the bathroom as well as robotic applications insofar they are intended for private household applications.

In a preferred embodiment the home appliance is a home appliance for preparing, storing and/or processing food. Preferably, the home appliance is then selected from a group consisting of stoves, ovens, baking ovens, microwaves, hobs, cooker hoods, refrigerators, freezers, coffee machines, plant oil cookers, kitchen machines and kitchen devices, barbeque devices, steam cookers and robotic applications for preparing, storing and/or processing food.

More preferably, the home appliance is selected from the group consisting of refrigerators, freezers, coffee machines, kitchen machines and kitchen devices.

Even more preferably, the home appliance is a refrigerator and/or a freezer.

In another preferred embodiment the home appliance is a water bearing home appliance. Preferably, the home appliance is then selected from a group consisting of laundry machines such as washing machines, washer dryers or dryers, dishwashers, steam irons, electrical small instantaneous water heaters, devices for heating and storing water for the kitchen and the bathroom, water boilers, as well as water bearing robotic applications.

More preferably, the home appliance is selected from the group consisting of washing machines, washer dryers, dryers or dishwashers.

Even more preferably, the home appliance is a washing machine or a washer dryer.

The term "(hetero)polyoxometalate", as used herein, encompasses the (hetero)polyoxometalates of formulae (I), (II), (III), (IV) and (V) (and sub-formulae thereof as described herein).

In the heteropolyoxometalates described herein, the "heteroatom" is phosphorus, silicon, germanium, aluminum or boron. If phosphorus, silicon, germanium, aluminum or boron are not included, the term "polyoxometalate" is appropriate for the designation of the species.

All (hetero)polyoxometalates described herein have molybdenum or tungsten oxide subunits which can be replaced in parts by titanium, vanadium, manganese, iron, cobalt, nickel or chromium oxide subunits.

The term "antimicrobial properties", as used herein, refers to the ability of the (hetero)polyoxometalates of formulae (I), (II), (III), (IV) and (V) (and sub-formulae thereof as described herein) to kill microorganisms.

The term "microorganisms", as referred to herein, include bacteria, viruses, fungi and algae. Preferably, the term "microorganisms", as used herein, refers to bacteria.

The term "biofilm", as used herein, refers to an assembly of microorganisms wherein cells stick to each other on the surface of a substrate in or on a home appliance. The term "growth of biofilm" or "biofilm growth", as used herein, refers to the microorganism built-up adhering to a surface of a substrate in or on a home appliance.

The term "reduction of the growth of a biofilm", as used herein, refers to the decrease, preferably the inhibition, of the microorganism built-up on a long term scale, i.e. at least 3 years, on the surface of a substrate in or on a home appliance comprising at least one (hetero)polyoxometalate as described herein compared to the surface of a substrate in or on a home appliance not comprising at least one (hetero)polyoxometalate as described herein.

The abbreviation "wt.-%" or "w/w", as used herein, means "weight percentage" and refers to the weight amount of a compound in relation to the (total) weight of a composition of compounds or of a substrate used in a home appliance if nothing else is explicitly stated or obvious under the circumstances.

It is understood that the cation(s) A is/are selected such that in the (hetero)polyoxometalate the charge of the compound of the Formula (I), (II), (III), (IV) and (V) is zero. For achieving this either the number and/or charge of cation(s) A can be altered and/or the charge is balanced by one or more further cations and/or anions.

Generally, polyoxometalates based on molybdenum (Mo) or tungsten (W) are known in the art, in particular as Keggin-type heteropolyoxometalate anions $[XZ_{12}O_{40}]^{n-}$ wherein the central heteroatom (X) can be e.g. phosphorus $(P^{5+})$, silicon $(Si^{4+})$, germanium $(Ge^{4+})$, aluminum $(Al^{3+})$, boron $(B^{3+})$ etc.. Further, in the Keggin-type polyoxometalate based on molybdenum or tungsten a number (1 or more) of molybdenum or tungsten atoms can be replaced by titanium atoms (e.g. $Ti^{4+}$), vanadium atoms (e.g. $V^{5+}$), nickel atoms (e.g. $Ni^{2+}$), iron atoms (e.g. $Fe^{3+}$), cobalt atoms (e.g. $Co^{2+}$ or $Co^{3+}$), zinc atoms $(Zn^{2+})$, chromium atoms (e.g. $Cr^{2+}$ or $Cr^{3+}$), manganese atoms (e.g. $Mn^{2+}$) etc.

Another example of polyoxometalate anions is the Wells-Dawson species $[X_2Z_{18}O_{62}]^{n-}$. This heteropolyoxometalate anion contains two heteroatoms (X), which can e.g. be phosphorus $(P^{5+})$, silicon $(Si^{4+})$, germanium $(Ge^{4+})$, aluminum $(Al^{3+})$, boron $(B^{3+})$ and 18 molybdenum or tungsten atoms (Z). Further, in the Wells-Dawson-type polyoxometalate based on tungsten one or more tungsten atoms may be replaced e.g. by vanadium atoms (e.g. $V^{5+}$).

Further well-known structures are the Venturello structure $[XZ_4U_{24}]^n$ and the Anderson structure $[XZ_6O_{24}]^{n-}$ with Z being tungsten $(W^{6+})$ or molybdenum $(Mo^{6+})$ and X being a heteroatom such as phosphorus $(P^{+5})$.

Structures of the formulae $[Z_6O_{19}]^{n-}$ are known as Lindquist structures with Z being $(W^{6+})$ or molybdenum $(Mo^{6+})$.

$(NH_4)_{14}Na[P_5W_{30}O_{100}]$ is known as the Preyssler-type heteropolyoxometalate.

Generally, these (hetero)polyoxometalate anions are known in the art, and the negative charge of the known anions is typically counterbalanced by cations like $Li^+$, $Na^+$, $K^+$ or $NH_4^+$ which provide solubility of the resulting salts in water.

In a preferred embodiment, the present invention relates to the use as described herein of (hetero)polyoxometalates of formulae (I), (II), (III), (IV) and (V), wherein in formulae (I), (II), (III), (IV) and (V) Z = W. With o = 0, this embodiment covers (hetero)polyoxometalates of the following formulae (I'), (II'), (III') and (V'):

$$[A_n]^{m+}[XM_qW_{r-q}O_s]^{m-} \qquad (I'),$$

wherein

m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
r = 11 or 12,
s = 37, 38, 39 or 40,
when r = 11, q = 0, 1, 2, 3, 4, 5, 6, 7, 8 or 9,

when r = 12, q = 0, 1, 2, 3, 4, 5, 6, 7, 8, 9 or 10, and
when r = 12, s = 40, X = P and q is 0, 1, 2 or 3, M is not V;

$$[A_n]^{m+}[XM_qW_{t-q}O_{24}]^{m-} \qquad (II'),$$

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
t = 4 or 6,
when t = 6, q = 0, 1, 2, 3 or 4,
when t = 4, q = 0, 1 or 2, and
when t = 4 and X = P, q is 1, 2 or 3;

$$[A_n]^{m+}[X_pM_qW_{6-q-p}O_{19}]^{m-} \qquad (III'),$$

wherein
m- is the negative charge of the (hetero)polyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
p = 0 or 1, and
q = 0, 1, 2 or 3
when p = 0, M is not V;

$$[A_n]^{m+}[X_2M_qW_{18-q}O_{62}]^{m-} \qquad (IV'),$$

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
q = 0, 1, 2, 3, 4, 5, 6, 7, 8, 9, 10, 11, 12, 13, 14, 15 or 16, and
when X = P, q is 1, 2 or 3;

$$[A_n]^{m+}[X_5M_qW_{30-q}O_{110}]^{m-} \qquad (V'),$$

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
q = 0, 1, 2, 3, 4, 5, 6, 7, 8, 9, 10, 11, 12, 13 ,14, 15, 16, 17, 18, 19, 20, 21, 22, 23, 24, 25, 26 or 27.

In Formula (I'), r is preferably 12, s is preferably 40 and q is preferably 0, 1, 2, or 3, thus resulting in a heteropolyoxometalate of the formula $[A_n]^{m+}(XM_qW_{12-q}O_{40})^{m-}$.

In Formula (II'), when t = 6, q is preferably 0, resulting in a heteropolyoxometalate of the formula $[A_n]^{m+}[XW_6O_{24}]^{m-}$.

In Formula (II'), when t = 4, q is preferably 0, resulting in a heteropolyoxometalate of the formula

$[A_n]^{m+}[XW_4O_{24}]^{m-}$.

In Formula (III'), p and q are preferably 0, resulting in a polyoxometalate of the formula $[A_n]^{m+}[W_6O_{19}]^{m-}$.

In Formula (IV'), q is preferably 0, 1, 2 or 3, and more preferably 0, resulting in a heteropolyoxometalate of the formula $[A_n]^{m+}[X_2W_{18}O_{62}]^{m-}$.

In Formula (V'), q is preferably 0, 1, 2, 3, and more preferably 0, resulting in a heteropolyoxometalate of the formula $[A_n]^{m+}[X_5W_{30}O_{110}]^{m-}$.

In a further preferred embodiment, the present invention relates to the use as described herein of (hetero)polyoxometalates of formulae (I), (II), (III), (IV), and (V), wherein in formulae (I), (II), (III), (IV), and (V) Z = Mo. With o = 0, this embodiment covers (hetero)polyoxometalates of the following formulae (I"), (II"), (III"), (IV") and (V"):

$$[A_n]^{m+}[XM_qMo_{r-q}O_s]^{m-} \qquad (I"),$$

wherein

m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
r = 11 or 12,
s = 37, 38, 39 or 40,
when r = 11, q = 0, 1, 2, 3, 4, 5, 6, 7, 8 or 9,
when r = 12, q = 0, 1, 2, 3, 4, 5, 6, 7, 8, 9 or 10, and
when r = 12, s = 40, X = P and q is 0, 1, 2 or 3, M is not V;

$$[A_n]^{m+}[XM_qMo_{t-q-o}O_{24}]^{m-} \qquad (II"),$$

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
t = 4 or 6,
when t = 6, q = 0, 1, 2, 3 or 4,
when t = 4, q = 0, 1 or 2, and
when t = 4 and X = P, q is 1, 2 or 3;

$$[A_n]^{m+}[X_pM_qMo_{6-q-p}O_{19}]^{m-} \qquad (III"),$$

wherein
m- is the negative charge of the (hetero)polyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
p = 0 or 1, and
q = 0, 1, 2 or 3
when p = 0, M is not V;

$$[A_n]^{m+}[X_2M_qMo_{18-q}O_{62}]^{m-} \qquad (IV"),$$

wherein
m- is the negative charge of the heteropolyoxometalate anion,
m+ is the positive charge of the cation(s) A,

lm-l = lm+l

n is the number of cation(s) A required to provide the positive charge m+,

X is selected from P, Si, Ge, Al and B,

M is selected from Ti, V, Mn, Fe, Co, Ni, Zn and Cr,

q = 0, 1, 2, 3, 4, 5, 6, 7, 8, 9, 10, 11, 12, 13, 14, 15 or 16, and

when X = P, q is 1, 2 or 3;

$$[A_n]^{m+}[X_5M_qMo_{30-q}O_{110}]^{m-} \qquad (V''),$$

wherein

m- is the negative charge of the heteropolyoxometalate anion,

m+ is the positive charge of the cation(s) A,

lm-l = lm+l

n is the number of cation(s) A required to provide the positive charge m+,

X is selected from P, Si, Ge, Al and B,

M is selected from Ti, V, Mn, Fe, Co, Ni, Zn and Cr,

q = 0, 1, 2, 3, 4, 5, 6, 7, 8, 9, 10, 11, 12, 13 ,14, 15, 16, 17, 18, 19, 20, 21, 22, 23, 24, 25, 26 or 27.

In Formula (I''), r is preferably 12, s is preferably 40 and q is preferably 0, 1, 2, or 3, thus resulting in a heteropolyoxometalate of the formula $[A_n]^{m+}[XM_qMo_{12-q}O_{40}]^{m-}$.

In Formula (II''), when t = 6, q is preferably 0, resulting in a heteropolyoxometalate of the formula $[A_n]^{m+}[XMo_6O_{24}]^{m-}$.

In Formula (II''), when t = 4, q is preferably 0, resulting in a heteropolyoxometalate of the formula $[A_n]^{m+}[XMo_4O_{24}]^{m-}$.

In Formula (III''), p and q are preferably 0, resulting in a polyoxometalate of the formula $[A_n]^{m+}[Mo_6O_{19}]^{m-}$.

In Formula (IV''), q is preferably 0, 1, 2 or 3, and more preferably 0, resulting in a heteropolyoxometalate of the formula $[A_n]^{m+}[X_2Mo_{18}O_{62}]^{m-}$.

In Formula (V''), q is preferably 0, 1, 2, 3, and more preferably 0, resulting in a heteropolyoxometalate of the formula $[A_n]^{m+}[X_5Mo_{30}O_{110}]^{m-}$.

In a further preferred embodiment, the present invention relates to the use as described herein of (hetero)polyoxometalates of formulae (I), (II), (III), (IV), and (V), wherein in formulae (I), (II), (III), (IV), and (V) Z = W and Z' = Mo and o = 1, thus covering (hetero)polyoxometalates of the following formulae (I'''), (II'''), (III'''), (IV''') and (V'''):

$$[A_n]^{m+}[XM_qW_{r-q-1}MoO_s]^{m-} \qquad (I'''),$$

wherein

m- is the negative charge of the heteropolyoxometalate anion,

m+ is the positive charge of the cation(s) A,

lm-l = lm+l

n is the number of cation(s) A required to provide the positive charge m+,

X is selected from P, Si, Ge, Al and B,

M is selected from Ti, V, Mn, Fe, Co, Ni, Zn and Cr,

r = 11 or 12,

s = 37, 38, 39 or 40,

when r = 11, q = 0, 1, 2, 3, 4, 5, 6, 7, 8 or 9,

when r = 12, q = 0, 1, 2, 3, 4, 5, 6, 7, 8, 9 or 10, and

when r = 12, s = 40, X = P and q is 0, 1, 2 or 3, M is not V;

$$[A_n]^{m+}[XM_qW_{t-q-1}MoO_{24}]^{m-} \qquad (II'''),$$

wherein

m- is the negative charge of the heteropolyoxometalate anion,

m+ is the positive charge of the cation(s) A,

lm-l = lm+l

n is the number of cation(s) A required to provide the positive charge m+,

X is selected from P, Si, Ge, Al and B,

M is selected from Ti, V, Mn, Fe, Co, Ni, Zn and Cr,

t = 4 or 6,
when t = 6, q = 0, 1, 2, 3 or 4,
when t = 4, q = 0, 1 or 2, and
when t = 4 and X = P, q is 1, 2 or 3;

$$[A_n]^{m+}[X_pM_qW_{5-q-p}MoO_{19}]^{m-} \qquad (III''')$$

wherein
m- is the negative charge of the (hetero)polyoxometalate anion,
m+ is the positive charge of the cation(s) A,
Im-I = Im+I
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
p = 0 or 1, and
q = 0, 1, 2 or 3;

$$[A_n]^{m+}[X_2M_qW_{17-q}MoO_{62}]^{m-} \qquad (IV''')$$

wherein
m- is the negative charge of the heteropolyoxometalate anion,
m+ is the positive charge of the cation(s) A,
Im-I = Im+I
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13, 14, 15 or 16, and
when X = P, q is 1, 2 or 3;

$$[A_n]^{m+}[X_5M_qW_{29-q}MoO_{110}]^{m-} \qquad (V''')$$

wherein
m- is the negative charge of the heteropolyoxometalate anion,
m+ is the positive charge of the cation(s) A,
Im-I = Im+I
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13 ,14, 15, 16, 17, 18, 19, 20, 21, 22, 23, 24, 25, 26 or 27;

In Formula (I'''), r is preferably 12, s is preferably 40 and q is preferably 0, 1, 2, or 3, thus resulting in a heteropolyoxometalate of the formula $[A_n]^{m+}[XM_qW_{11-q}MoO_{40}]^{m-}$.
In Formula (II'''), when t = 6, q is preferably 0, resulting in a heteropolyoxometalate of the formula $[A_n]^{m+}[XW_5MoO_{24}]^{m-}$.
In Formula (II'''), when t = 4, q is preferably 0, resulting in a heteropolyoxometalate of the formula $[A_n]^{m+}[XW_3MoO_{24}]^{m-}$.
In Formula (III'''), p and q are preferably 0, resulting in a polyoxometalate of the formula $[A_n]^{m+}[W_5MoO_{19}]^{m-}$.
In Formula (IV'''), q is preferably 0, 1, 2 or 3, and more preferably 0, resulting in a heteropolyoxometalate of the formula $[A_n]^{m+}[X_2W_{17}MoO_{62}]^{m-}$.
In Formula (V'''), q is preferably 0, 1, 2, 3, and more preferably 0, resulting in a heteropolyoxometalate of the formula $[A_n]^{m+}[X_5W_{29}MoO_{110}]^{m-}$.
In a further preferred embodiment, the present invention relates to the use as described herein of (hetero)polyoxometalates of formulae (I), (II), (III), (IV), and (V), wherein in formulae (I), (II), (III), (IV), and (V) X is selected from P, Si and Al.

It is also preferred that in formulae (I'), (II'), (III'), (IV'), and (V') X is selected from P, Si and Al.

It is furthermore preferred that in formulae (I"), (II"), (III"), (IV"), and (V") X is selected from P, Si and Al.

It is furthermore preferred that in formulae (I‴), (II‴), (III‴), (IV‴), and (V‴) X is selected from P, Si and Al.

In a further preferred embodiment, the present invention relates to the use as described herein of (hetero)polyoxometalates of formulae (I), (II), (III), (IV), and (V), wherein in formulae (I), (II), (III), (IV), and (V) M is selected from Co, Ti and V.

It is also preferred that in formulae (I'), (II'), (III'), (IV'), and (V') M is selected from Co, Ti and V.

It is furthermore preferred that in formulae (I"), (II"), (III"), (IV"), and (V") M is selected from Co, Ti and V.

It is furthermore preferred that in formulae (I‴), (II‴), (III‴), (IV‴), and (V‴) M is selected from Co, Ti and V.

In a further preferred embodiment, the present invention relates to the use as described herein of (hetero)polyoxometalates of formulae (I), (II), (III), (IV), and (V), wherein in formulae (I), (II), (III), (IV), and (V) X = P or Si and M = V.

It is also preferred that in formulae (I'), (II'), (III'), (IV'), and (V') X = P or Si and M = V.

It is furthermore preferred that in formulae (I"), (II"), (III"), (IV"), and (V") X = P or Si and M = V.

It is furthermore preferred that in formulae (I‴), (II‴), (III‴), (IV‴), and (V‴) X = P or Si and M = V.

In an alternative preferred embodiment, the present invention relates to the use as described herein of (hetero)polyoxometalates of formulae (I), (II), (III), (IV), and (V), wherein in formulae (I), (II), (III), (IV), and (V) X = P or Si and M = Ti.

It is also preferred that in formulae (I'), (II'), (III'), (IV'), and (V') X = P or Si and M = Ti.

It is furthermore preferred that in formulae (I"), (II"), (III"), (IV"), and (V") X = P or Si and M = Ti.

It is furthermore preferred that in formulae (I‴), (II‴), (III‴), (IV‴), and (V‴) X = P or Si and M = Ti.

In a further alternative preferred embodiment, the present invention relates to the use as described herein of (hetero)polyoxometalates of formulae (I), (II), (III), (IV), and (V), wherein in formulae (I), (II), (III), (IV), and (V) X = P or Si and M = Co.

It is also preferred that in formulae (I'), (II'), (III'), (IV'), and (V') X = P or Si and M = Co.

It is furthermore preferred that in formulae (I"), (II"), (III"), (IV"), and (V") X = P or Si and M = Co.

It is furthermore preferred that in formulae (I‴), (II‴), (III‴), (IV‴), and (V‴) X = P or Si and M = Co.

In a further alternative preferred embodiment, the present invention relates to the use as described herein of (hetero)polyoxometalates of formulae (I), (II), (III), (IV), and (V), wherein in formulae (I), (II), (III), (IV), and (V) (I), (II), (III), (IV), and (V) X = Al and M = Co, V or Ti.

It is also preferred that in formulae (I'), (II'), (III'), (IV'), and (V') X = Al and M = Co, V or Ti.

It is furthermore preferred that in formulae (I"), (II"), (III"), (IV"), and (V") X = Al and M = Co, V or Ti.

It is furthermore preferred that in formulae (I‴), (II‴), (III‴), (IV‴), and (V‴) X = Al and M = Co, V or Ti.

Particularly preferred (hetero)polyoxometalates for use in simultaneously imparting antimicrobial properties to a surface of a substrate in or on a home appliance and reducing, preferably inhibiting, the growth of a biofilm on the surface of the substrate in or on a home appliance are the following species:

$A_n[SiVW_{11}O_{40}]$, $A_n[SiV_2W_{10}O_{40}]$, $A_n[SiV_3W_9O_{40}]$, $A_n[SiV_4W_8O_{40}]$, $A_n[SiVMo_{11}O_{40}]$, $A_n[SiV_2Mo_{10}O_{40}]$, $A_n[SiV_3Mo_9O_{40}]$, $A_n[SiV_4Mo_3O_{40}]$, $A_n[PV_6Mo_6O_{40}]$, $A_n[PV_5Mo_7O_{40}]$, $A_n([PV_8Mo_4O_{40}]$, $A_n[PCoW_{11}O_{39}]$, $A_n[AlVW_{11}O_{40}]$, $A_n[SiVW_{10}MoO_{40}]$, based on heteropolyoxometalates of Formula (I);

$A_n[PW_6O_{24}]$, $A_n[PMo_6O_{24}]$, $A_n[SiW_6O_{24}]$, $A_n[SiMo_6O_{24}]$, $A_n[AlW_6O_{24}]$, $A_n[AlMo_6O_{24}]$, $A_n[SiW_4O_{24}]$, $A_n[SiMo_4O_{24}]$, $A_n[AlW_4O_{24}]$, $A_n[AlMo_4O_{24}]$, based on heteropolyoxometalates of Formula (II);

$A_n[W_6O_{19}]$ and $A_n[Mo_6O_{19}]$, based on polyoxometalates of Formula (III);

$A_n[Si_2W_{18}O_{62}]$, $A_n[Si_2Mo_{18}O_{62}]$, based on heteropolyoxometalates of Formula (IV);

$A_n[P_5W_{30}O_{110}]$, $A_n[Si_5W_{30}O_{110}]$, $A_n[Al_5W_{30}O_{110}]$, $A_n[P_5Mo_{30}O_{110}]$, $A_n[Si_5Mo_{30}O_{110}]$, $A_n[Al_5Mo_{30}O_{110}]$, based on heteropolyoxometalates of Formula (V).

It is understood that in these species, the cation(s) A is/are selected such that in the (hetero)polyoxometalate the charge is zero. For achieving this either the number and/or charge of cation(s) A can be altered and/or the charge is balanced by one or more further cations, e.g. selected from the ammonium ion ($NH_4^+$), alkaline metal ions such as $Li^+$, $Na^+$, $K^+$, $Rb^+$, $Cs^+$, and alkaline earth metal ions such as $Be^{2+}$, $Mg^{2+}$, $Ca^{2+}$, $Sr^{2+}$, $Ba^{2+}$.

Furthermore, it is possible that the (hetero)polyoxometalate anions comprise neutral ligands such as, e.g., $H_2O$, leading to structures such as, e.g., $A_n[AlMn(H_2O)W_{11}O_{30}]$

In a further preferred embodiment of the present invention, A comprises at least one cation selected from the group consisting of quaternary ammonium cations of Formula (VI), quaternary phosphonium cations of Formula (VII) and tertiary sulfonium cations of Formula (VIII)

$$R^1R^2R^3R^4N^+ \qquad (VI)$$

$$R^1R^2R^3R^4P^+ \qquad (VII)$$

$$R^1R^2R^3S^+ \qquad (VIII)$$

wherein residues $R^1$, $R^2$, $R^3$ and $R^4$ are independently selected from the group consisting of C1 to C80 hydrocarbons, polymers, and heterocyclic compounds, wherein at least two of the residues $R^1$, $R^2$, $R^3$ and, if present, $R^4$ are hydrocarbons $\geq$ C8, i.e. with more than 8 carbon atoms, and optionally at least one of the residues $R^1$, $R^2$, $R^3$ and, if present, $R^4$ are part of a ring or form a ring together with the nitrogen, phosphor or sulfur atom. A is however not a quaternary ammonium cation when $[XM_qZ_{r-q-o}Z'_oO_s]^m$ is $[SiV_3W_9O_{40}]^7$.
These cations have antimicrobial properties contributing to the antimicrobial properties of the overall (hetero)polyoxometalate species and to its ability to reduce the biofilm growth on the surface of a substrate in or on a home appliance.

The presence of a quaternary ammonium cation of Formula (VI), a quaternary phosphonium cation of Formula (VII) or a tertiary sulfonium cation of Formula (VIII) also renders the (hetero)polyoxometalates as described herein less soluble or even insoluble in water, especially, when at least two of the residues $R^1$, $R^2$, $R^3$ and, if present, $R^4$ are hydrocarbons $\geq$ C8. Furthermore, when at least two of the residues $R^1$, $R^2$, $R^3$ and, if present, $R^4$ are hydrocarbons $\geq$ C8, it was found that cell penetration of the respective cations is enhanced favoring the position of N+, P+ and/or S+ in the cellular membrane leading to the destruction of said membrane. Thus, a better antimicrobial activity and reduction of biofilm growth on the surface of a substrate in or on a home appliance can be achieved.

Preferably, the solubility of the (hetero)polyoxometalates of the present invention is below 10 mg/l, preferably below 1 mg/ml in water at 20°C, more preferably below 0.1 mg/ml water, in particular below 0.01 mg/ml water.

The lower solubility or even insolubility for aqueous liquids also contributes to the long term efficacy of the (hetero)polyoxometalate as described herein regarding the reduction of the biofilm growth on the surface of a substrate in or on a home appliance and antimicrobial activity since they are not washed out the substrate used in a home appliance over time upon exposure to water or moisture.

The C1 to C80 hydrocarbons encompass branched or straight, saturated or unsaturated, substituted or unsubstituted alkyl groups, aryl groups or heteroaryl groups. These residues thus can also be waxes or wax-like.

Examples for these C1 to C80 hydrocarbons include methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, C30 alkyl, C31 alkyl, C32 alkyl, C33 alkyl, C34 alkyl, C35 alkyl, C36 alkyl, C37 alkyl, C39 alkyl, C40 alkyl, C41 alkyl, C42 alkyl, C43 alkyl, C44 alkyl, C45 alkyl, C46 alkyl, C47 alkyl, C48 alkyl, C49 alkyl, C50 alkyl, C51 alkyl, C52 alkyl, C53 alkyl, C54 alkyl, C55 alkyl, C56 alkyl, C57 alkyl, C58 alkyl, C59 alkyl, C60 alkyl, C61 alkyl C62 alkyl, C63 alkyl, C64 alkyl, C65 alkyl, C66 alkyl, C67 alkyl, C68 alkyl, C69 alkyl, C70 alkyl, C71 alkyl, C72 alkyl, C73 alkyl, C74 alkyl, C75 alkyl, C75 alkyl, C76 alkyl, C77 alkyl, C78 alkyl, C79 alkyl, and C80 alkyl residues. These C1 to C80 hydrocarbons are preferably straight.

Preferred are C1 to C60 hydrocarbons including methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, C30 alkyl, C31 alkyl, C32 alkyl, C33 alkyl, C34 alkyl, C35 alkyl, C36 alkyl, C37 alkyl, C39 alkyl, C40 alkyl, C41 alkyl, C42 alkyl, C43 alkyl, C44 alkyl, C45 alkyl, C46 alkyl, C47 alkyl, C48 alkyl, C49 alkyl, C50 alkyl, C51 alkyl, C52 alkyl, C53 alkyl, C54 alkyl, C55 alkyl, C56 alkyl, C57 alkyl, C58 alkyl, C59 alkyl and C60 alkyl residues. These C1 to C60 hydrocarbons are preferably straight.

More preferred are C1 to C40 hydrocarbons including methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, C30 alkyl, C31 alkyl, C32 alkyl, C33 alkyl, C34 alkyl, C35 alkyl, C36 alkyl, C37 alkyl, C39 alkyl and C40 alkyl residues. These C1 to C40 hydrocarbons are preferably straight.

More preferred are C1 to C30 hydrocarbons including methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, and C30 alkyl residues. These C1 to C30 hydrocarbons are preferably straight.

More preferred are C2 to C25 hydrocarbons including ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, and C25 alkyl. These C2 to C25 are preferably straight.

Particularly preferred are C4 to C20 hydrocarbons including butyl, propyl, hexyl, heptyl, octyl, nonyl, decyl, undecyl, dodecyl, tridecyl, tetradecyl, pentadecyl, hexadecyl, heptadecy, octadecyl, nonadecyl, and eicosanyl residues. These C4 to C20 hydrocarbons are preferably straight.

The hydrocarbons $\geq$ C8 encompass branched or straight, saturated or unsaturated, substituted or unsubstituted alkyl groups, aryl groups or heteroaryl groups. These residues thus can also be waxes or wax-like.

Particularly preferred, however are hydrocarbons, such as e.g. octyl, nonyl, decyl, undecyl or dodecyl..

Suitable polymers include, for example, polymers comprising cationic side chains, such as phosphonium-containing cationic poly(styrene) polymers, hydroxy exchange membranes comprising quaternary ammonium hydroxide or quaternary phosphonium hydroxide functional groups, poly(vinylamine) derivatives as described for example in EP 0 580 078 A1, polymeric phosphonium ionomers, as described for example in WO 94/10214, poly(alkyl- and aryl)p-phenoxy-phenylsulfonium salts, poly(acrylamide-co-diallyl-dimethylammonium) and poly(diallyldimethylammonium).

The ring formed together with the nitrogen, phosphor or sulfur atom may, for example, be a three, four, five, six or seven-membered ring which may be saturated or unsaturated and which may contain one or more further heteroatoms, such as oxygen, nitrogen, phosphor or sulfur atoms. If the ring is unsaturated and if a double bond is present at the nitrogen, phosphor or sulfur atom of the ammonium, phosphonium or sulfonium cation, then one of $R^1$, $R^2$ $R^3$ or $R^4$ may be absent. Examples of suitable rings include aziridinium, thiiranium, azetidinium, thietium, pyrrolidinium, tetrathydrothiophenium, pyrrolium, thiophenium, piperidinium, tetrahydrothiopyranium, pyridinium, thiopyrylium, hexamethyleniminium, hexamethylensulfidium, azatropilidenium, thiotropilidenium, pyrazolium, imidazolium, benzimidazolium, imidazolinium, indolium, chinolinium, isochinolinium, purinium, pyrimidinium, oxazolium, thiazolium, thiazinium, triazines, thiazoles, thiazolines, piperidines, oxazolidines, oxazolidones, guanine derivatives, cytosine derivatives, adenine derivatives, thymine derivatives, cyclic amino-acid and peptide derivatives, cyclic biguanide derivatives, cyclic guanidine derivatives, as well as the phosphorous analogs of these ring systems.

The rings may be substituted by one or more hydrocarbon residues, in particular $C_1$ to $C_{12}$ alkyl or aryl (in particular phenyl) $C_1$ to $C_6$ alkyl residues. Suitable cations containing a ring are for example 1-butyl-3-methylimidazolium, 1-butyl-2,3-dimetylimidazolium, 1-methyl-3-octylimidazolium, 1-hexadecyl-3-methylimidazolium, 1,3-didecyl-3-methylimidazolium and 1-benzyl-3-methylimidazolium.

Preferred quaternary ammonium cations are e.g. tetramethylammonium, tetraethylammonium, tetrapropylammonium, tetrabutylammonium, tetrapentylammonium tetrahexylammonium, tetraheptylammonium tetraoctylammonium cation, tetranonylammonium cation, tetradecylammonium cation, tetraundecylammonium cation, tetradodecylammonium cation, tetratridecylammonium cation, tetratetradecylammonium cation, tetrapentadecylammonium cation, methyltributylammonium cation, methyltripentylammonium cation, methyltrihexylammonium cation, methyltriheptylammonium cation, methyltrioctylammonium cation, methyltrinonylammonium cation, methyltridecylammonium cation, methyltriundecylammonium cation, methyltridodecylammonium cation, methyltritridecylammonium cation, methyltritetradecylammonium cation, tributylhexylammonium cation, tributylheptylammonium cation, tributyloctylammonium cation, tributylnonylammonium cation, tributyldecylammonium cation, tributylundecylammonium cation, tributyldodecylammonium cation, tributyltridecylammonium cation, tributyltetradecylammonium cation, tributylpentadecylammonium cation, tributylhexadecylammonium cation, trihexyltetradecylammonium cation and trihexylhexadecylammonium cation, in particular tetrabutylammonium cation, tetrahexylammonium cation, methyltrioctylammonium cation, tributyltetradecylammonium cation.

Preferred quaternary phosphonium cations are e.g. tetrapropylphosphonium cation, tetrabutylphosphonium cation, tetrapentylphosphonium cation, tetrahexylphosphonium cation, tetraheptylphosphonium cation, tetraoctylphosphonium cation, tetranonylphosphonium cation, tetradecylphosphonium cation, tetraundecylphosphonium cation, tetradodecylphosphonium cation, tetratridecylphospphonium cation, tetratetradecylphosphonium cation, methyltrioctylphosphonium cation, tributyltetradecylphosphonium cation, tributyldodecylphosphonium cation, trihexyltetradecylphosphonium cation, trihexylhexadecylphosphonium cation in particular tetrabutylphosphonium cation, tetrahexylphosphonium cation, methyltributylphosphonium cation, methyltripentylphosphonium cation, methyltrihexylphosphonium cation, methyltriheptylphosphonium cation, methyltrioctylphosphonium cation, methyltrinonylphosphonium cation, methyltridecylphosphonium cation, tributylpentylphosphonium cation, tributylhexylphosphonium cation, tributylheptylphosphonium cation, tributyloctylphosphonium cation, tributylnonylphosphonium cation, tributyldecylphosphonium cation, tributylundecylphosphonium cation, tributyldodecylphosphonium cation, tributyltridecylphosphonium cation, tributyltetradecylphosphonium cation, tributylpentadecylphosphonium cation, tributylhexadecylphosphonium cation, trihexylheptylphosphonium cation, trihexyloctylphosphonium cation, trihexylnonylphosphonium cation, trihexyldecylphosphonium cation, trihexylundecylphosphonium cation, trihexyldodecylphosphonium cation, trihexyltridecylphosphonium cation, trihexyltetradecylphosphonium cation, trihexylpentadecylphosphonium cation and trihexylhexadecylphosphonium cation.

Preferred tertiary sulfonium cations are e.g. tributylsulfonium cation, tripentylsulfonium cation, trihexylsulfonium cation, triheptylsulfonium cation, trioctylsulfonium cation, methyldioctylsulfonium cation and dibutyltetradecylsulfonium cation, in particular tributylsulfonium cation and trihexylsulfonium cation.

Further suitable ammonium, phosphonium and sulfonium cations and the preparation thereof are known in the art.

In a preferred embodiment of the present invention, in the (hetero)polyoxometalate A comprises at least one cation selected from the group consisting of quaternary ammonium cations and quaternary phosphonium cations.

In a more preferred embodiment of the present invention, in the (hetero)polyoxometalate A comprises at least one, preferably at least two quaternary ammonium cations.

In an alternative more preferred embodiment of the present invention, in the (hetero)polyoxometalate A comprises at least one, preferably at least two quaternary phosphonium cations.

Particularly preferred (hetero)polyoxometalates are e.g.:

$[(CH_3(CH_2)_3)_4N]_5[SiVW_{11}O_{40}]$, $[(CH_3(CH_2)_3)_4N]_6[SiV_2W_{10}O_{40}]$, $[(CH_3(CH_2)_3)_3P(C_{14}H_{29})]_5[SiVW_{11}O_{40}]$, $[(CH_3(CH_2)_3)_3P(C_{14}H_{29})]_6[SiV_2W_{10}O_{40}]$ $[(CH_3(CH_2)_3)_3P(C_{14}H_{29})]_7[SiV_3W_9O_{40}]$, $[(CH_3(CH_2)_3)_3P(C_{14}H_{29})]_8[SiV_4W_8O_{40}]$, $[(CH_3(CH_2)_3)_3P(C_{14}H_{29})]_8[SiV_4Mo_8O_{40}]$, $[(CH_3(CH_2)_7)_4N]_5[SiVMo_{11}O_{40}]$, $[(CH_3(CH_2)_7)_3N(CH_3)]_6[SiV_2Mo_{10}O_{40}]$, $[(CH_3(CH_2)_3)_3P((CH_2)_{13}CH_3)]_7[SiV_3Mo_9O_{40}]$, $[(CH_3(CH_2)_3)_4N]_{11}[PV_8Mo_4O_{40}]$, $[(CH_3(CH_2)_7)_3N(CH_3)]_5[PCoW_{11}O_{39}]$, $[(CH_3(CH_2)_3)_3P((CH_2)_{13}CH_3)]_6[AlVW_{11}O_{40}]$, $[(CH_3(CH_2)_3)_4N]_5[SiVW_{10}MoO_{40}]$, $[(CH_3(CH_2)_3)_3P(C_{14}H_{29})]_9[PV_6Mo_6O_{40}]$, $[(CH_3(CH_2)_3)_3P(C_{14}H_{29})]_8[PV_5Mo_7O_{40}]$, based on (hetero)polyoxometalates of Formula (I);

$[(CH_3(CH_2)_3)_4N]_7[PW_6O_{24}]$, $[(CH_3(CH_2)_7)_4N]_7[PMO_6O_{24}]$, $[(CH_3(CH_2)_7)_3N(CH_3)]_8[SiW_6O_{24}]$, $[(CH_3(CH_2)_3)_3P((CH_2)_{13}CH_3)]_8[SiMo_6O_{24}]$, $[(CH_3(CH_2)_3)_4N]_9[AlW_6O_{24}]$, $[(CH_3(CH_2)_3)_4N]_9[AlMO_6O_{24}]$, $[(CH_3(CH_2)_5)_4N]_{20}[SiW_4O_{24}]$, $[(CH_3(CH_2)_7)_4N]_{20}[SiMo_4O_{24}]$, $[(CH_3(CH_2)_7)_3N(CH_3)]_{21}[AlW_4O_{24}]$, $[(CH_3(CH_2)_3)_3P((CH_2)_{13}CH_3)]_{21}[AlMo_4O_{24}]$, based on (hetero)polyoxometalates of Formula (II);

$[(CH_3(CH_2)_3)_4N]_2[W_6O_{19}]$ and $[(CH_3(CH_2)_3)_3P((CH_2)_{13}CH_3)]_2[Mo_6O_{19}]$, based on (hetero)polyoxometalates of Formula (III);

$[(CH_3(CH_2)_7)_3N(CH_3)]_8[Si_2W_{18}O_{62}]$, $[(CH_3(CH_2)_3)_3P((CH_2)_{13}CH_3)]_8[Si_2Mo_{18}O_{62}]$, based on (hetero)polyoxometalates of Formula (IV);

$[(CH_3(CH_2)_3)_4N]_{15}[P_5W_{30}O_{110}]$, $[(CH_3(CH_2)_7)_3N(CH_3)]_{20}[Si_5W_{30}O_{110}]$, $[(CH_3(CH_2)_5)_4N]_{15}[P_5Mo_{30}O_{110}]$, $[(CH_3(CH_2)_5)_4N]_{20}[Si_5Mo_{30}O_{110}]$, based on (hetero)polyoxometalates of Formula (V).

These compounds are particularly suitable for simultaneously imparting antimicrobial properties to a surface of a substrate in or on a home appliance and reducing, particularly inhibiting, the growth of a biofilm on the surface of the substrate in or on a home appliance because they are particularly suitable for activating molecular oxygen and producing ROS, thereby reducing, preferably inhibiting, the growth of biofilm on a substrate surface in or on a home appliance and maintaining the microbial efficacy of the heteropolyoxometalates on a long term time scale (at least 3 years).

In a preferred embodiment of the present invention, the (hetero)polyoxometalate is of Formula (II) and A comprises at least one quaternary ammonium cation of Formula (VI). It was found that especially the (hetero)polyoxometalates of Formula (II), wherein A comprises at least one quaternary ammonium cation of Formula (VI), show superior activity in the generation of reactive oxygen species and thus an improved antimicrobial activity and ability to reduce biofilm growth.

In a moreover preferred embodiment of the present invention, the (hetero)polyoxometalate is of Formula (II) and A comprises at least one quaternary phosphonium cation of Formula (VII). It was found that especially the (hetero)polyoxometalate of Formula (II), wherein A comprises at least one quaternary phosphonium cation of Formula (VII), remain stable and antimicrobially active even under high temperature conditions with the temperature being equal to or above 60°C.

Preferably, if the (hetero)polyoxometalate is of Formula (II) ) and A either comprises at least one quaternary ammonium cation of Formula (VI) or at least one phosphonium cation of Formula (VII), the (hetero)polyoxometalate is selected from the group of particularly preferred (hetero)polyoxometalates based on Formula (II) as stated above.

The (hetero)polyoxometalates of formulae (I), (II), (III), (IV) and (V) and sub-formulae thereof as described herein can be prepared according to known processes. Often, heteropolyoxometalates can be prepared from minimally toxic compounds such as e.g. $WO_4^{2-}$, $MoO_4^{2-}$, $PO_4^{3-}$, $SiO_3^{2-}$ $VO_3^-$ (see I.A. Weinstock et al., Selective Transition-Metal Catalysis of Oxygen Delignification Using Water-Soluble Salts of Polyoxometalate (POM) Anions, Part I. Chemical Principles and Process Concepts, Holzforschung 1998, 52, 304-310). General synthesis methods are also disclosed in EP 2 765 136 A1 and WO 2014/122225 A1. Syntheses of exemplary heteropolyoxometalates as disclosed herein are described in the examples.

The present invention furthermore relates to the use of a substrate in or on a home appliance comprising a (hetero)polyoxometalate as defined above, or a mixture thereof, wherein the substrate is a polymer body, or a polymer coating.

A polymer body, as referred to herein, is any object used in or on a home appliance comprising at least one polymer.

The term "coating" or "polymer coating", as used herein, is any liquid composition that upon administration to a substrate used in a home appliance in a thin layer converts into a solid film for decorative or protective purposes. The term "coating" thus refers to the liquid composition before administration to a substrate used in a home appliance and to the solid film obtained after administration of the liquid coating composition to the substrate used in a home appliance, and the skilled person will understand from the context, which state of the coating is meant.

According to the invention the coating is a polymer coating. Typically, polymers for polymer coatings are selected from the group consisting of acrylate resins, polyurethane resins, silicon resins, polyester resins, alkyd resins, epoxy resins, phenolic resins, and urea or amine based resins.

In a preferred embodiment of the present invention, the polymer body or the polymer coating comprises a thermoplastic, an elastomer, a thermoplastic elastomer, a duroplast, or a mixture thereof.

The term "thermoplastic", as used herein, refers to a polymer that becomes pliable or moldable above a specific temperature and solidifies upon cooling. Examples for thermoplastic polymers include but are not limited to polyacrylates, acrylonitrile-butadiene-styrenes, polyamides such as nylon, polyacetic acid, polybenzimidazole, polycarbonate, polyether sulfone, polyetherether ketone, polyetherimide, polyethylene, polyphenylene oxide, polyphenylen sulfide, polypropylene, polystyrene, polyvinylchloriode, polyethyleneterephthalate, polyurethane, polyester and polytetrafluoroethylene (e.g. Teflon).

The term "elastomer", as used herein, refers to a polymer with viscoelasticity (having both viscosity and elasticity). Examples for elastomers include but are not limited to unsaturated rubbers such as natural polyisoprene (natural rubber), synthetic polyisoprene, polybutadiene, chloroprene rubber, butyl rubber, styrene-butadiene rubber, (hydrogenated) nitrile rubber, saturated rubbers such as ethylene propylene rubber, ethylene propylene diene rubber, epichlorohydrin rubber, polyacrylic rubber, silicone, silicone rubber, fluorosilicone rubber, fluoro- and perfluoroelastomers, and ethylene-vinyl acetate.

The term "thermoplastic elastomer", as used herein, refers to a class of copolymers or a physical mix of polymers which consists of materials with both thermoplastic and elastomeric properties. Examples for thermoplastic elastomers include but are not limited to styrenic block copolymers, polyolefin blends, elastomeric alloys, thermoplastic polyurethanes, thermoplastic copolyester, and thermoplastic polyamides.

The term "duroplast", as used herein, refers to a polymer which is no longer pliable after curing. Examples for duroplasts include but are not limited to aminoplasts, phenoplasts, epoxy resins, polyacrylates, polyurethanes, polyesters, urea formaldehyde resins, melamine formaldehyde resins, and phenol formaldehyde resins.

In the present invention, the polymer body or the polymer coating comprises a polymer selected from the group consisting of polypropylene, polyethylene, polyethyleneterephthalate, polyester, polyamide, polyurethane, polyacrylate, polycarbonate, polystyrene, polyimides, polymethacrylates, polyoxoalkylenes, poly(phenylene oxides), polyvinylesters, polyvinylethers, polyvinylidene chloride, acrylonitrile-butadiene-styrene, natural and synthetic polyisoprene, polybutadiene, chloroprene rubber, styrene-butadiene rubber, tetrafluoroethylene, silicone, acrylate resins, polyurethane resins, silicone resins, polyester resins, alkyd resins, epoxy resins, phenolic resins, and urea or amine based resins, or a mixture thereof.

In a particularly preferred embodiment of the present invention, the substrate used in or on a home appliance is a polymer body and the polymer is polypropylene.

The substrate used in or on a home appliance comprises the (hetero)polyoxometalate or the mixture thereof in an amount of 1 to 15 wt.-%, and preferably in an amount of 10 wt.-%, each based on the total weight of the substrate.

The surface of a substrate used in or on a home appliance wherein this amount of (hetero)polyoxometalate is present, is particularly suitable for imparting antimicrobial properties to the surface of the substrate in or on a home appliance and reduces, preferably inhibits, at the same time the growth of a biofilm on the surface of the substrate in or on a home appliance. Moreover, since the (hetero)polyoxometalates as described herein have a low solubility in water, they are not washed out of the substrate used in or on a home appliance upon exposure to water and moisture which guarantees a long term efficacy of the (hetero)polyoxometalate in providing antimicrobial properties to the surface of the substrate in or on a home appliance and reducing, preferably inhibiting, the growth of a biofilm.

The (hetero)polyoxometalates as described herein can be incorporated into a substrate used in or on a home appliance as described above, into a polymer body or a polymer coating, by means of techniques known to the person skilled in the art.

The incorporation of the (hetero)polyoxometalate into a polymer body can e.g. be achieved by mixing the (hetero)polyoxometalate with the polymer in order to obtain a dispersion or a compound of (hetero)polyoxometalate and polymer. Said dispersion or compound can then e.g. be submitted to injection molding or extrusion for forming the polymer body.

Alternatively, the (hetero)polyoxometalate can be mixed with the monomers before polymerization is carried out. After polymerization, the resulting polymer can e.g. be submitted to compounding processes, pressureless processing techniques (e.g. casting, dipping, coating, foaming) or compression molding, rolling and calendaring, extrusion, blow molding or injection molding processes or drawing, thermoforming or printing for forming the polymer body.

The polymer body as described herein can also be obtained by drylaid, airlaid, spunlaid/meltblown or wetlaid processes, in particular if the polymer body is for use as fiber material or non-woven material.

The incorporation of the (hetero)polyoxometalate into a polymer coating composition can e.g. be achieved by mixing the (hetero)polyoxometalate, which e.g. can be used as a powder, with a coating solution in order to obtain a suspension of the (hetero)polyoxometalate and the coating solution. Said suspension can be stored before usage, i.e. application of the coating solution in its intended use.

In a particularly preferred embodiment of the present invention, the substrate comprises at least one (hetero)polyoxometalate of Formula (II), wherein A comprises at least one quaternary phosphonium cation of Formula (VII). It was found that especially (hetero)polyoxometalates of Formula (II), wherein A comprises at least one quaternary phosphonium cation of Formula (VII) exhibit superior temperature stability. This is in particular advantageous, if high temperatures are applied in the manufacturing of the substrate. For example, this would be the case if the substrate is a polymer body and the at least one (hetero)polyoxometalate would be incorporated during an injection molding process.

The substrate used in a home appliance comprising the (hetero)polyoxometalate as described herein can be used for self-cleaning, stripping, disinfecting, self-sanitizing, biocidal, antimicrobial, and/or deodorizing purposes and/or for the decomposition and/or degradation of organic materials.

In particular, the substrate used in a home appliance comprising the (hetero)polyoxometalate as described herein can be used for the disinfection of an aqueous media.

The present invention furthermore relates to a home appliance wherein a substrate as defined herein is used in or on said home appliance, and wherein the substrate comprises at least one (hetero)polyoxometalate as defined herein.

The term "home appliance" as used herein, refers to devices which are intended to be used in private household applications, also including communication- and data processing devices, which are suitable und intended to interact with the devices for private household applications. In particular, the following household applications are included: stoves, ovens, baking ovens, microwaves, hobs, cooker hoods, dishwashers, laundry machines, dryers, refrigerators, freezers, vacuum cleaners, coffee machines, water boilers, plant oil cookers, irons, hair dryers, shavers, kitchen machines and kitchen devices, barbeque devices, steam cookers, electrical small instantaneous water heaters, devices for heating and storing water for the kitchen and the bathroom as well as robotic applications insofar they are intended for private household applications.

In a preferred embodiment, however, the home appliance is a home appliance for preparing, storing and/or processing food. Preferably, the home appliance is then selected from a group consisting of stoves, ovens, baking ovens, microwaves, hobs, cooker hoods, refrigerators, freezers, coffee machines, plant oil cookers, kitchen machines and kitchen devices, barbeque devices, steam cookers and robotic applications for preparing, storing and/or processing food.

More preferably, the home appliance is then selected from the group consisting of refrigerators, freezers, coffee machines, kitchen machines and kitchen devices.

Even more preferably, the home appliance is then a refrigerator and/or a freezer.

In another preferred embodiment the home appliance is a water bearing home appliance. Preferably, the home appliance is then selected from a group consisting of laundry machines such as washing machines, washer dryers or dryers, dishwashers, steam irons, electrical small instantaneous water heaters, devices for heating and storing water for the kitchen and the bathroom, water boilers, as well as water bearing robotic applications.

More preferably, the home appliance is then selected from the group consisting of washing machines, washer dryers, dryers or dishwashers.

Even more preferably, the home appliance is then a washing machine or a washer dryer.

The main advantages of the application of the described technology are that (hetero)polyoxometalates can work for years without additional activation or power consumption as a catalytic system. Thus, the radicals, which sanitize the home appliance, can be produced easily. For the user of the home appliance, this technology is very convenient as no manual work is involved and no refill of spent cleaning chemicals is necessary.

The application of any of the (hetero)polyoxometalates as described herein in or on a home appliance has to be carried out on the surface to be sanitized or within reach of the radicals. For example, the tub of a washing machine can be made at least partly with one of the (hetero)polyoxometalates comprising substrates or it can be coated or covered at least partly with a substrate. Thus the growth in this - from a hygienically point of view - critical area of the appliance is inhibited. Like this, all relevant surfaces of the various home appliances can be covered or elsewise be applied with a (hetero)polyoxometalate in order to improve the hygienic situation.

Further applications of this technology in home appliances include e.g. the use of the (hetero)polyoxometalates for sanitizing and disinfection purposes in:

- all water-carrying parts of a home appliance, such as the tub and the detergent dispenser of a washing machine, a dryer, the condensate container, water supply and discharge lines, pumps, including housing, valves;

- all inner surfaces of a refrigerator used as home appliance;
- all surfaces of coffee machines used as home appliance and other parts thereof such as water tanks, water pipes, filter holder, drip tray, milk frother, pulp container and all containers in which temporary garbage, waste or spent / spoiled food
- all surfaces of furnaces used as home appliance;
- all surfaces of range hoods used as home appliance, especially the sieves and filter mats thereof, possibly also for the treatment of air;
- all surfaces of a microwaves used as home appliance;
- all surfaces of steamers used in home appliances;
- all surfaces of cookers and hobs used in home appliances;
- all surfaces of steam stations and steam irons used as home appliances, in particular the surface of a water tank of these home appliances;
- all surfaces of humidifiers, air conditioners and air conditioners used as home appliances.

Examples

Example 1: Synthesis of $[(C_4H_9)_3P(C_{14}H_{29})]_5[SiVW_{11}O_{40}]$ (It-1)

$K_8[\alpha\text{-}SiW_{11}O_{39}]\times13H_2O$ was dissolved in hot water under stirring. After cooling, the pH was adjusted to 5.8 with HCl. $NaVO_3$ solution was slowly added under stirring, while the pH was adjusted to 5.8 with HCl, and stirred for 5 min. Next, the pH was adjusted to 2.0 using HCl and the solution was stirred at the same pH for 45 min. The resulting solution was filtered and TBTDP-Cl ($[(C_4H_9)_3P(C_{14}H_{29})]Cl$) was added. The yellow precipitate was separated by suction filtration, washed with $H_2O$ and dried under vacuum.

Example 2: Synthesis of $[(C_4H_9)_3P(C_{14}H_{29})]_7[SiV_3W_9O_{40}]$ (It-2)

$NaVO_3$ (0.7 g, 6.0 mmol) and 5.0 g of $Na10[\alpha\text{-}SiW_9O_{34}]\times xH_2O$ (2.0 mmol) were mixed as dry powders and added to 50 ml of $H_2O$ at room temperature (25°C). The solution was stirred vigorously during 30 min and then 6 M HCL was added dropwise to bring the pH to 1.5, and a clear wine red solution developed. The resulting solution was filtered and 15.0 g of TBTDP-Cl ($[(C_4H_9)_3P(C_{14}H_{29})]Cl$) was added. The orange gel was separated by removing the upper aqueous layer and washed 5 times with 50 ml of $H_2O$.

Comparative Example 1: Synthesis of $[(C_4H_9)_3P(C_{14}H_{29})]_7[PVW_{11}O_{40}]$ (It-4)

$H_3PW_{12}O_{40}$ was dissolved in $H_2O$ and solid $Li_2CO_3$ was added to pH 4.9. The solution of $NaVO_3$ was added with stirring. HCl was added dropwise to pH 2 and the solution was heated to 60 °C for 10 min before returning it to room T. Additional HCl was added to bring the mixture back to pH 2 and the solution was reheated to 60 °C. The resulting solution was filtered and TBTDP-Cl ($[(C_4H_9)_3P(C_{14}H_{29})]Cl$) was added. The yellow precipitate was separated by suction filtration, washed with $H_2O$ and dried under vacuum.

Example 3: Antimicrobial surface activity

The compounds It-1, It-2 and It-4, respectively obtained in Examples 1 and 2 and Comparative Example 1, were used in the preparation of test pieces for the determination of the antimicrobial surface activity of these compounds.

Samples of the compounds It-1, It-2, and It-4 were respectively incorporated into an acrylate-based composition (said acrylate composition being designated in the following as "A1220", consisting of 57% (w/w) WorléeCryl (Worléechemie), 14% (w/w) Desmodur N75 BA (Bayer) and 29% (w/w) acrylate thinner (Gräsolin), each based on the total weight of the acrylate composition), resulting in (hetero)polyoxometalate-functionalized compositions with a 30% (w/w) loading of It-1 (Sample 1), It-2 (Sample 2), and It-4 (Sample 3), based on the total weight of the composition.

Polypropylene (PP) pieces (ca. 5 × 5 cm) were respectively coated with a layer of the hetero)polyoxometalate-functionalized compositions according to Sample 1, Sample 2 and Sample 3, and the resulting coated pieces were cured in the open air. For each sample, 6 polypropylene test pieces were prepared.

6 further polypropylene pieces were coated with a neutral layer of A1220 (i.e. not comprising a (hetero)polyoxometalate) as the blind sample (BS).

The test microorganism (Staphylococcus aureus, DSMZ No. 799) was prepared by growth in a liquid culture medium (trypticase soy broth, TSB). The suspension of test microorganism was standardized by dilution in a nutrient broth.

The surfaces of Samples 1 to 3 and the blind sample (BS) were inoculated with microorganism and then the microbial inoculum was covered with a thin, sterile film in order to spread the inoculum, prevent it from evaporating and to ensure close contact with the antimicrobial surface. Microbial concentrations of Samples 1 to 3 and the blind sample (BS) were determined at "time zero" (t = 0) by elution, followed by dilution and plating. Samples 1 to 4 were then allowed to incubate undisturbed in a humid environment for 24 hours at 36 °C and 90% relative humidity. After incubation, microbial concentrations were determined by elution, followed by dilution and plating.

The antimicrobial activity value (R) of Samples 1 to 3 was determined via:

$$R = \log (B/A) - \log (C/A),$$

wherein:

A is the average of the CFU value of the untreated surface, after t = 0 hrs,
B is the average of the CFU value of the untreated surface, after 24 hrs incubation,
C is the average of the CFU value of the active surface, after 24 hrs incubation,

wherein CFU is the colony forming unit.

The results for Samples 1 to 3 and the blind sample (BS) are shown in Table 1 (based on the average of 6 test pieces for each sample).

**Table 1**

| Sample # | Coating of PP | t = 0 | t = 24 hrs | Antimicrobial activity value (R) |
|---|---|---|---|---|
| | | CFU/test surface | | |
| 1 | 30% It-1 in A1220 | - | 17 | 5,44 |
| 2 | 30% It-2 in A1220 | - | < 10 | > 5,66 |
| 3 | 30% It-4 in A1220 | - | 410 | 4,05 |
| BS | neutral A1220 | $1,3 \times 10^7$ | $4,6 \times 10^6$ | - |

The surfaces of both Samples 1 to 2 showed a high antimicrobial activity. Particularly high killing rates were achieved with Sample 2 (It-2). In this sample, no microorganisms were detectable after 24 hours. Sample 1 (It-1) also showed a high value of antimicrobial activity. Compared to Samples 1 and 2, the antimicrobial activity of Sample 3 was significantly lower.

Example 4: Reduction of the growth of biofilm

The compound It-2, obtained in Example 2, was used in the preparation of an heteropolyoxometalate-functionalized active coating consisting of the acrylic polymer composition A1220 as described above, and 10% (w/w) of It-2 (Sample 1), based on the total weight of the active coating.

2 polypropylene (PP) test pieces (2.5 × 5 cm) were prepared by coating one part of the test piece with the active coating according to Sample 1. The other part of the test piece remained free of active coating. 2 further polypropylene pieces without coating were used as the blind sample (BS).

The test microorganism (Staphylococcus aureus (S. aureus), DSMZ No. 799) was prepared by growth in a liquid culture medium (trypticase soy broth, TSB). The suspension of test microorganism was standardized by dilution in a nutrient broth. The polypropylene test pieces according to Sample 1 were immersed in the S. aureus containing nutrient solution in a petri dish, respectively.

The samples prepared above are summarized in the following Table 2:

**Table 2**

| Sample # | Coating of PP | Description |
|---|---|---|
| 1 | 10% It-2 in A1220 | 2 PP test pieces (2.5 x 5 cm), one part with active coating |
| BS | - | 2 PP test pieces (2.5 x 5 cm) without active coating |

After 1 week, one of the polypropylene test pieces of Sample 1 and of the blind sample (BS) was taken out of the petri dish, dried at 20°C, and submitted to SEM (scanning electron microscope) spectroscopy. The results after 1 week for Sample 1 comparing the coated part (left SEM image) with the uncoated part of the polypropylene test piece (right SEM image) are shown in Fig. 1a. The result for the blind sample (BS) as determined by SEM spectroscopy after 1 week is shown in Fig. 2a.

The nutrient solution in the petri dishes was replaced by a fresh nutrient solution containing the microorganism, and the polypropylene test pieces according to Sample 1 and the blind sample (BS) were allowed to rest for another week.

After 2 weeks in total, a further polypropylene test piece of Sample 1 and the blind sample (BS) was taken out of the petri dish, dried at 20°C, and submitted to SEM spectroscopy. The results after 2 weeks for Sample 1 comparing the coated part (left SEM image) with the uncoated part of the test piece (right SEM image) are shown in Fig. 1b. The result for the blind sample (BS) as determined by SEM spectroscopy after 2 weeks is shown in Fig. 2b.

Fig. 1a shows the growth of S. aureus biofilm on both the coated part of the test piece (left SEM image of Fig. 1a) and the uncoated part of the test piece (right SEM image of Fig. 1a) after 1 week. Fig. 1b no longer shows S. aureus biofilm on both the coated part of the test piece (left SEM image of Fig. 1b) and the uncoated part of the test piece (right SEM image of Fig. 1b) after 2 weeks. The contamination shown in Fig. 1b is mold fungus.

Sample 1 thus shows a reduction of the growth of biofilm after 2 weeks exposure to a nutrient medium containing S. aureus microorganism. The effect of the active coating also spreads into the uncoated vicinity of the active coating.

The blind sample (BS) does not show this decrease in the biofilm growth after 2 weeks exposure to a nutrient medium containing S. aureus microorganism (compare Fig. 2a, taken after 1 week to Fig. 2b, taken after 2 weeks.

**Claims**

1. Use of at least one (hetero)polyoxometalate for simultaneously imparting antimicrobial properties to a surface of a substrate in or on a home appliance and reducing the growth of a biofilm on the surface of the substrate in or on a home appliance for at least 3 years, wherein the at least one (hetero)polyoxometalate is of the formula (I), (II), (III), (IV) and/or (V):

$$[A_n]^{m+}[XM_qZ_{r-q-o}Z'_oO_s]^{m-} \qquad (I),$$

wherein

m- is the negative charge of the heteropolyoxometalate anion,
m+ is the positive charge of the cation(s) A,
Im-I = Im+I
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
r = 11 or 12,
o = 0 or 1,
s = 37, 38, 39 or 40,
when r = 11, q = 0, 1, 2, 3, 4, 5, 6, 7, 8 or 9,
when r = 12, q = 0, 1, 2, 3, 4, 5, 6, 7, 8, 9 or 10, and
when r = 12, s = 40, X = P and q is 0, 1, 2 or 3, M is not V;

$$[A_n]^{m+}[XM_qZ_{t-q-o}Z'_oO_{24}]^{m-} \qquad (II),$$

wherein
m- is the negative charge of the heteropolyoxometalate anion,
m+ is the positive charge of the cation(s) A,
Im-I = Im+I
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
t = 4 or 6,
o = 0 or 1,
when t = 6, q = 0, 1, 2, 3 or 4,
when t = 4, q = 0, 1 or 2, and

when t = 4 and X = P, q is 1, 2 or 3;

$$[A_n]^{m+}[X_pM_qZ_{6-q-p-o}Z'_oO_{19}]^{m-} \qquad (III),$$

wherein
m- is the negative charge of the (hetero)polyoxometalate anion,
m+ is the positive charge of the cation(s) A,
lm-l = lm+l
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
p = 0 or 1, and
q = 0, 1, 2 or 3,
when p = 0, M is not V;

$$[A_n]^{m+}[X_2M_qZ_{18-q-o}Z'_oO_{62}]^{m-} \qquad (IV),$$

wherein
m- is the negative charge of the heteropolyoxometalate anion,
m+ is the positive charge of the cation(s) A,
lm-l = lm+l
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13, 14, 15 or 16, and
when X = P, q is 1, 2 or 3;

$$[A_n]^{m+}[X_5M_qZ_{30-q-o}Z'_oO_{110}]^{m-} \qquad (V),$$

wherein
m- is the negative charge of the heteropolyoxometalate anion,
m+ is the positive charge of the cation(s) A,
lm-l = lm+l
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13 14, 15, 16, 17, 18, 19, 20, 21, 22, 23, 24, 25, 26 or 27;
wherein A is selected from one or more cations and comprises at least one cation selected from the group consisting of quaternary ammonium cations, quaternary phosphonium cations and tertiary sulfonium cations, with the proviso that A is not a quaternary ammonium cation when $[XM_qZ_{r-q-o}Z'_oO_s]^m$ is $[SiV_3W_9O_{40}]^{7-}$, and wherein the substrate is a polymer body or polymer coating comprising a polymer selected from the group consisting of polypropylene, polyethylene, polyethyleneterephthalate, polyester, polyamide, polyurethane, polyacrylate, polycarbonate, polystyrene, polyimides, polymethacrylates, polyoxoalkylenes, poly(phenylene oxides), polyvinylesters, polyvinylethers, polyvinylidene chloride, acrylonitrile-butadiene-styrene, natural and synthetic polyisoprene, polybutadiene, chloroprene rubber, styrene-butadiene rubber, tetrafluoroethylene, silicone, acrylate resins, polyurethane resins, silicone resins, polyester resins, alkyd resins, epoxy resins, phenolic resins, and urea or amine based resins, or a mixture thereof, ethylene propylene rubber and ethylene propylene diene rubber , and wherein the substrate comprises the at least one (hetero)polyoxometalate in an amount of 1 to 15 wt.-%, based on the total weight of the substrate.

2. Use according to claim 1, wherein in formulae (I), (II), (III), (IV), and (V) Z = W.

3. Use according to claim 1, wherein in formulae (I), (II), (III), (IV), and (V) Z = Mo.

4. Use according to any of claims 1 to 3, wherein in formulae (I), (II), (III), (IV), and (V) X is selected from P, Si and Al.

5. Use according to any of claims 1 to 4, wherein in formulae (I), (II), (III), (IV), and (V) M is selected from Co, Ti and V.

6. Use according to any of claims 1 to 5, wherein in formulae (I), (II), (III), (IV), and (V) X = P or Si and M = V.

7. Use according to claims 1 to 5, wherein in formulae (I), (II), (III), (IV), and (V) X = P or Si and M = Ti.

8. Use according to claims 1 to 5, wherein in formulae (I), (II), (III), (IV), and (V) X = P or Si and M = Co.

9. Use according to claims 1 to 5, wherein in formulae (I), (II), (III), (IV), and (V) X = Al and M = Co, V or Ti.

10. Use according to any of the preceding claims, wherein A comprises at least one cation selected from the group consisting of quaternary ammonium cations of Formula (VI), quaternary phosphonium cations of Formula (VII) and tertiary sulfonium cations of Formula (VIII)

$$R^1R^2R^3R^4N^+ \qquad (VI)$$

$$R^1R2R^3R^4P^+ \qquad (VII)$$

$$R^1R^2R^3S^+ \qquad (VIII)$$

wherein residues $R^1$, $R^2$, $R^3$ and $R^4$ are independently selected from the group consisting of C1 to C80 hydrocarbons, polymers and heterocyclic compounds, wherein at least two of the residues $R^1$, $R^2$, $R^3$ and, if present, $R^4$ are hydrocarbons $\geq$ C8 and optionally at least one of the residues $R^1$, $R^2$, $R^3$ and, if present, $R^4$ are part of a ring or form a ring together with the nitrogen, phosphor or sulfur atom.

11. Use according to any of the preceding claims, wherein the (hetero)polyoxometalate is of Formula (II) and A comprises at least one quaternary ammonium cation of Formula (VI).

12. Use according to any of claims 1 to 10, wherein the (hetero)polyoxometalate is of Formula (II) and A comprises at least one quaternary phosphonium cation of Formula (VII).

13. Use of a substrate in or on a home appliance comprising at least one (hetero)polyoxometalate as defined in any of the preceding claims, wherein the substrate is a polymer body or a polymer coating comprising a polymer selected from the group consisting of polypropylene, polyethylene, polyethyleneterephthalate, polyester, polyamide, polyurethane, polyacrylate, polycarbonate, polystyrene, polyimides, polymethacrylates, polyoxoalkylenes, poly(phenylene oxides), polyvinylesters, polyvinylethers, polyvinylidene chloride, acrylonitrile-butadiene-styrene, natural and synthetic polyisoprene, polybutadiene, chloroprene rubber, styrene-butadiene rubber, tetrafluoroethylene, silicone, acrylate resins, polyurethane resins, silicone resins, polyester resins, alkyd resins, epoxy resins, phenolic resins, and urea or amine based resins, or a mixture thereof, ethylene propylene rubber and ethylene propylene diene rubber.

14. Use of a substrate in or on a home appliance according to claim 13 wherein the at least one (hetero)polyoxometalate is the (hetero)polyoxometalate according to claim 11.

15. Use of a substrate in or on a home appliance according to claim 13, wherein the at least one (hetero)polyoxometalate is the (hetero)polyoxometalate according to claim 12.

16. Use of a substrate in or on a home appliance according to any of claims 13 to 15, wherein the substrate is a polymer body, and wherein the polymer is polypropylene.

17. Home appliance, wherein a substrate as defined in any of claims 13 to 16 is used in or on said home appliance, and wherein the substrate comprises at least one (hetero)polyoxometalate as defined in any of claims 1 to 12.

**Patentansprüche**

1. Verwendung von mindestens einem (Hetero)polyoxometallat zur gleichzeitigen Übertragung antimikrobieller Eigenschaften auf einer Oberfläche eines Substrats in oder auf einem Haushaltsgerät und zur Verringerung des Wachstums eines Biofilms auf der Oberfläche des Substrats in oder auf einem Haushaltsgerät für mindestens 3 Jahre, wobei das mindestens eine (Hetero)polyoxometallat die Formel (I), (II), (III), (IV) und/oder (V) aufweist:

$$[A_n]^{m+}[XM_qZ_{r-q-o}Z'_oO_s]^{m-} \qquad (I),$$

wobei

m- die negative Ladung des Heteropolyoxometallat-Anions ist,
m+ die positive Ladung des Kations/der Kationen A ist,
$|m-| = |m+|$,
n die Anzahl an Kation(en) A ist, die erforderlich ist, um die positive Ladung m+ bereitzustellen,
Z und Z' unabhängig ausgewählt sind aus W und Mo,
X ausgewählt ist aus P, Si, Ge, Al und B,
M ausgewählt ist aus Ti, V, Mn, Fe, Co, Ni, Zn und Cr,
r = 11 oder 12,
o = 0 oder 1,
s = 37, 38, 39 oder 40,
wenn r = 11, q = 0, 1, 2, 3, 4, 5, 6, 7, 8 oder 9,
wenn r = 12, q = 0, 1, 2, 3, 4, 5, 6, 7, 8, 9 oder 10, und
wenn r = 12, s = 40, X = P und q 0, 1, 2 oder 3 ist, M nicht V ist,

$$[A_n]^{m+}[XM_qZ_{t-q-o}Z'_oO_{24}]^{m-} \qquad (II),$$

wobei
m- die negative Ladung des Heteropolyoxometallat-Anions ist,
m+ die positive Ladung des Kations/der Kationen A ist,
$|m-| = |m+|$,
n die Anzahl an Kation(en) A ist, die erforderlich ist, um die positive Ladung m+ bereitzustellen,
Z und Z' unabhängig ausgewählt sind aus W und Mo,
X ausgewählt ist aus P, Si, Ge, Al und B,
M ausgewählt ist aus Ti, V, Mn, Fe, Co, Ni, Zn und Cr,
t = 4 oder 6,
o = 0 oder 1,
wenn t = 6, q = 0, 1, 2, 3 oder 4,
wenn t = 4, q = 0, 1 oder 2, und
wenn t = 4 und X = P, q 1, 2 oder 3 ist,

$$[A_n]^{m+}[X_pM_qZ_{6-q-p-o}Z'_oO_{19}]^{m-} \qquad (III),$$

wobei
m- die negative Ladung des (Hetero)polyoxometallat-Anions ist,
m+ die positive Ladung des Kations/der Kationen A ist,
$|m-| = |m+|$,
n die Anzahl an Kation(en) A ist, die erforderlich ist, um die positive Ladung m+ bereitzustellen,
Z und Z' unabhängig ausgewählt sind aus W und Mo,
X ausgewählt ist aus P, Si, Ge, Al und B,
M ausgewählt ist aus Ti, V, Mn, Fe, Co, Ni, Zn und Cr,
o = 0 oder 1,
p = 0 oder 1, und
q = 0, 1, 2 oder 3,
wenn p = 0, M nicht V ist,

$$[A_n]^{m+}[X_2M_qZ_{18-q-o}Z'_oO_{62}]^{m-} \qquad (IV),$$

wobei

m- die negative Ladung des Heteropolyoxometallat-Anions ist,

m+ die positive Ladung des Kations/der Kationen A ist,

|m-| = |m+|,

n die Anzahl an Kation(en) A ist, die erforderlich ist, um die positive Ladung m+ bereitzustellen,

Z und Z' unabhängig ausgewählt sind aus W und Mo,

X ausgewählt ist aus P, Si, Ge, Al und B,

M ausgewählt ist aus Ti, V, Mn, Fe, Co, Ni, Zn und Cr,

o = 0 oder 1,

q = 0, 1, 2, 3, 4, 5, 6, 7, 8, 9, 10, 11, 12, 13, 14, 15 oder 16, und

wenn X = P, q 1, 2 oder 3 ist,

$$[A_n]^{m+}[X_5M_qZ_{30-q-o}Z'_oO_{110}]^{m-} \qquad (V),$$

wobei

m- die negative Ladung des Heteropolyoxometallat-Anions ist,

m+ die positive Ladung des Kations/der Kationen A ist,

|m-| = |m+|,

n die Anzahl an Kation(en) A ist, die erforderlich ist, um die positive Ladung m+ bereitzustellen,

Z und Z' unabhängig ausgewählt sind aus W und Mo,

X ausgewählt ist aus P, Si, Ge, Al und B,

M ausgewählt ist aus Ti, V, Mn, Fe, Co, Ni, Zn und Cr,

o = 0 oder 1,

q = 0, 1, 2, 3, 4, 5, 6, 7, 8, 9, 10, 11, 12, 13, 14, 15, 16, 17, 18, 19, 20, 21, 22, 23, 24, 25, 26 oder 27;

wobei A ausgewählt ist aus einem oder mehreren Kationen und mindestens ein Kation ausgewählt aus der Gruppe bestehend aus quartären Ammoniumkationen, quartären Phosphoniumkationen und tertiären Sulfoniumkationen umfasst,

mit der Maßgabe, dass A kein quaternäres Ammoniumkation ist, wenn $[XM_qZ_{r-q-o}Z'_oO_s]^m$ $[SiV_3W_9PO_{40}]^{7-}$ ist, und wobei das Substrat ein Polymerkörper oder eine Polymerbeschichtung ist, umfassend ein Polymer, ausgewählt aus der Gruppe bestehend aus Polypropylen, Polyethylen, Polyethylenterephthalat, Polyester, Polyamid, Polyurethan, Polyacrylat, Polycarbonat, Polystyrol, Polyimiden, Polymethacrylaten, Polyoxoalkylenen, Poly(phenylenoxiden), Polyvinylestern, Polyvinylethern, Polyvinylidenchlorid, Acrylnitril-Butadien-Styrol, natürlichem und synthetischem Polyisopren, Polybutadien, Chloroprenkautschuk, Styrol-Butadien-Kautschuk, Tetrafluorethylen, Silikon, Acrylatharzen, Polyurethanharzen, Silikonharzen, Polyesterharzen, Alkydharzen, Epoxidharzen, Phenolharzen und Harzen auf Harnstoff- oder Aminbasis oder einer Mischung davon, Ethylen-Propylen-Kautschuk und Ethylen-Propylen-Dien-Kautschuk, und wobei das Substrat das mindestens eine (Hetero)polyoxometallat in einer Menge von 1 bis 15 Gew.-% basierend auf dem Gesamtgewicht des Substrats umfasst.

2.  Verwendung nach Anspruch 1, wobei in Formel (I), (II), (III), (IV) und (V) Z = W.

3.  Verwendung nach Anspruch 1, wobei in Formel (I), (II), (III), (IV) und (V) Z = Mo.

4.  Verwendung nach einem der Ansprüche 1 bis 3, wobei in Formel (I), (II), (III), (IV) und (V) X ausgewählt ist aus P, Si und Al.

5.  Verwendung nach einem der Ansprüche 1 bis 4, wobei in Formel (I), (II), (III), (IV) und (V) M ausgewählt ist aus Co, Ti und V.

6.  Verwendung nach einem der Ansprüche 1 bis 5, wobei in Formel (I), (II), (III), (IV) und (V) X = P oder Si und M = V.

7.  Verwendung nach einem der Ansprüche 1 bis 5, wobei in Formel (I), (II), (III), (IV) und (V) X = P oder Si und M = Ti.

8.  Verwendung nach einem der Ansprüche 1 bis 5, wobei in Formel (I), (II), (III), (IV) und (V) X = P oder Si und M = Co.

9.  Verwendung nach einem der Ansprüche 1 bis 5, wobei in Formel (I), (II), (III), (IV) und (V) X = Al und M = Co, V oder Ti.

10. Verwendung nach einem der vorhergehenden Ansprüche, wobei A mindestens ein Kation umfasst, ausgewählt aus der Gruppe bestehend aus quartären Ammoniumkationen der Formel (VI), quartären Phosphoniumkationen der Formel (VII) und tertiären Sulfoniumkationen der Formel (VIII)

$$R^1R^2R^3R^4N^+ \qquad (VI)$$

$$R^1R^2R^3R^4P^+ \qquad (VII)$$

$$R^3R^2R^3S^+ \qquad (VIII)$$

wobei die Reste $R^1$, $R^2$, $R^3$ und $R^4$ unabhängig ausgewählt sind aus der Gruppe bestehend aus C1- bis C80-Kohlenwasserstoffen, Polymeren und heterozyklischen Verbindungen, wobei mindestens zwei der Reste $R^1$, $R^2$, $R^3$ und, falls vorhanden, $R^4$ Kohlenwasserstoffe $\geq$ C8 sind und optional mindestens einer der Reste $R^1$, $R^2$, $R^3$ und, falls vorhanden, $R^4$ Teil eines Rings ist oder einen Ring zusammen mit dem Stickstoff-, Phosphor- oder Schwefelatom bildet.

11. Verwendung nach einem der vorhergehenden Ansprüche, wobei das (Hetero)polyoxometallat die Formel (II) aufweist und A mindestens ein quartäres Ammoniumkation der Formel (VI) umfasst.

12. Verwendung nach einem der Ansprüche 1 bis 10, wobei das (Hetero)polyoxometallat die Formel (II) aufweist und A mindestens ein quartäres Phosphoniumkation der Formel (VII) umfasst.

13. Verwendung eines Substrats in oder auf einem Haushaltsgerät, umfassend mindestens ein (Hetero)polyoxometallat wie in einem der vorhergehenden Ansprüche definiert, wobei das Substrat ein Polymerkörper oder eine Polymerbeschichtung ist, umfassend ein Polymer, ausgewählt aus der Gruppe bestehend aus Polypropylen, Polyethylen, Polyethylenterephthalat, Polyester, Polyamid, Polyurethan, Polyacrylat, Polycarbonat, Polystyrol, Polyimiden, Polymethacrylaten, Polyoxoalkylenen, Poly(phenylenoxiden), Polyvinylestern, Polyvinylethern, Polyvinylidenchlorid, Acrylnitril-Butadien-Styrol, natürlichem und synthetischem Polyisopren, Polybutadien, Chloroprenkautschuk, Styrol-Butadien-Kautschuk, Tetrafluorethylen, Silikon, Acrylatharzen, Polyurethanharzen, Silikonharzen, Polyesterharzen, Alkydharzen, Epoxidharzen, Phenolharzen und Harzen auf Harnstoff- oder Aminbasis oder einer Mischung davon, Ethylen-Propylen-Kautschuk und Ethylen-Propylen-Dien-Kautschuk.

14. Verwendung eines Substrats in oder auf einem Haushaltsgerät nach Anspruch 13, wobei das mindestens eine (Hetero)polyoxometallat das (Hetero)polyoxometallat nach Anspruch 11 ist.

15. Verwendung eines Substrats in oder auf einem Haushaltsgerät nach Anspruch 13, wobei das mindestens eine (Hetero)polyoxometallat das (Hetero)polyoxometallat nach Anspruch 12 ist.

16. Verwendung eines Substrats in oder auf einem Haushaltsgerät nach einem der Ansprüche 13 bis 15, wobei das Substrat ein Polymerkörper ist und wobei das Polymer Polypropylen ist.

17. Haushaltsgerät, wobei ein Substrat wie in einem der Ansprüche 13 bis 16 definiert in oder auf dem Haushaltsgerät verwendet wird und wobei das Substrat mindestens ein (Hetero)polyoxometallat wie in einem der Ansprüche 1 bis 12 definiert umfasst.

**Revendications**

1. Utilisation d'au moins un (hétéro)polyoxométalate pour conférer simultanément des propriétés antimicrobiennes à la surface d'un substrat dans ou sur un appareil domestique et réduire la croissance d'un biofilm à la surface du substrat dans ou sur un appareil domestique pendant au moins 3 ans. dans lequel le au moins un (hétéro)polyoxométalate est de formule (I), (II), (III), (IV) et/ou (V) :

$$[A_n]^{m+}[XM_qZ_{r-q-o}Z'_oO_s]^{m-} \qquad (I),$$

dans laquelle

    m- est la charge négative de l'anion hétéropolyoxométalate,
    m+ est la charge positive du ou des cations A,
    Im-I = Im+I

n est le nombre de cation(s) A requis pour fournir la charge positive m+,

Z et Z' sont indépendamment sélectionnés parmi W et Mo,

X est sélectionné parmi P, Si, Ge, Al et B,

M est sélectionné parmi Ti, V, Mn, Fe, Co, Ni, Zn et Cr,

r = 11 ou 12,

o = 0 ou 1,

s = 37, 38, 39 ou 40,

quand r = 11, q = 0, 1, 2, 3, 4, 5, 6, 7,8 ou 9,

quand r = 12, q = 0, 1, 2, 3, 4, 5, 6, 7, 8, 9 ou 10, et

quand r = 12, s = 40, X = P et q est 0, 1, 2 ou 3, M n'est pas V;

$$[A_n]^{m+}[XM_qZ_{t-q-o}Z'_oO_{24}]^{m-} \qquad (II),$$

dans laquelle

m- est la charge négative de l'anion hétéropolyoxométalate,

m+ est la charge positive du ou des cations A,

Im-I = Im+I

n est le nombre de cation(s) A requis pour fournir la charge positive m+,

Z et Z' sont indépendamment sélectionnés parmi W et Mo,

X est sélectionné parmi P, Si, Ge, Al et B,

M est sélectionné parmi Ti, V, Mn, Fe, Co, Ni, Zn et Cr,

t = 4 ou 6,

o = 0 ou 1,

quand t = 6, q = 0, 1, 2, 3 ou 4,

quand t = 4, q = 0, 1 ou 2, et

quand t = 4 et X = P, q est 1, 2 ou 3;

$$[A_n]^{m+}[X_pM_qZ_{6-q-p-o}Z'_oO_{19}]^{m-} \qquad (III),$$

dans laquelle

m- est la charge négative de l'anion (hétéro)polyoxométalate,

m+ est la charge positive du ou des cations A,

Im-I = Im+I

n est le nombre de cation(s) A requis pour fournir la charge positive m+,

Z et Z' sont indépendamment sélectionnés parmi W et Mo,

X est sélectionné parmi P, Si, Ge, Al et B,

M est sélectionné parmi Ti, V, Mn, Fe, Co, Ni, Zn et Cr,

o = 0 ou 1,

p = 0 ou 1, et

q = 0, 1, 2 ou 3,

quand p = 0, M n'est pas V;

$$[A_n]^{m+}[X_2M_qZ_{18-q-o}Z'_oO_{62}]^{m-} \qquad (IV),$$

dans laquelle

m- est la charge négative de l'anion hétéropolyoxométalate,

m+ est la charge positive du ou des cations A,

Im-I = Im+I

n est le nombre de cation(s) A requis pour fournir la charge positive m+,

Z et Z' sont indépendamment sélectionnés parmi W et Mo,

X est sélectionné parmi P, Si, Ge, Al et B,

M est sélectionné parmi Ti, V, Mn, Fe, Co, Ni, Zn et Cr,

o = 0 ou 1,

q = 0, 1, 2, 3, 4, 5, 6, 7, 8, 9, 10, 11, 12, 13, 14, 15 ou 16 et

quand X = P, q est 1, 2 ou 3;

$$[A_n]^{m+}[X_5M_qZ_{30-q-o}Z'_oO_{110}]^{m-} \qquad (V),$$

dans laquelle

m- est la charge négative de l'anion hétéropolyoxométalate,

m+ est la charge positive du ou des cations A,

Im-I = Im+I

n est le nombre de cation(s) A requis pour fournir la charge positive m+,

Z et Z' sont indépendamment sélectionnés parmi W et Mo,

X est sélectionné parmi P, Si, Ge, Al et B,

M est sélectionné parmi Ti, V, Mn, Fe, Co, Ni, Zn et Cr,

o = 0 ou 1,

q = 0, 1, 2, 3, 4, 5, 6, 7, 8, 9, 10, 11, 12, 13,14, 15, 16, 17, 18, 19, 20, 21, 22, 23, 24, 25, 26 ou 27 ;

dans laquelle A est sélectionné parmi un ou plusieurs cations et comprend au moins un cation sélectionné parmi le groupe composé des suivants : des cations ammonium quaternaire, des cations phosphonium quaternaire et des cations sulfonium tertiaire,

à condition que A n'est pas un cation ammonium quaternaire quand $[XM_qZ_{r-q-o}Z'_oO_s]^m$ est $[SiV_3W_9O_{40}]^{7-}$, et dans lequel le substrat est un corps de polymère ou un revêtement de polymère comprenant un polymère sélectionné parmi le groupe composé des suivants : polypropylène, polyéthylène, poly(téréphtalate d'éthylène), polyester, polyamide, polyuréthane, polyacrylate, polycarbonate, polystyrène, polyimides, polyméthacrylates, polyoxoalkylènes, poly(oxydes de phénylène), polyvinylesters, polyvinyléthers, chlorure de polyvinylidène, acrylonitrile-butadiène-styrène, polyisoprène naturel et synthétique, polybutadiène, caoutchouc de chloroprène, caoutchouc de styrène-butadiène, tétrafluoroéthylène, silicone, résines acrylate, résines de polyuréthane, résines de silicone, résines de polyester, résines alkyde, résines époxy, résines phénoliques et résines à base d'urée ou d'amine, ou un mélange de ceux-ci, caoutchouc d'éthylène propylène et caoutchouc d'éthylène propylène diène et dans lequel le substrat comprend le au moins un (hétéro)polyoxométalate dans une quantité allant de 1 à 15% en poids, sur base du poids total du substrat.

2. Utilisation selon la revendication 1, dans laquelle, dans les formules (I), (II), (III), (IV) et (V), Z = W.

3. Utilisation selon la revendication 1, dans laquelle, dans les formules (I), (II), (III), (IV) et (V), Z = Mo.

4. Utilisation selon l'une quelconque des revendications 1 à 3, dans laquelle, dans les formules (I), (II), (III), (IV) et (V), X est sélectionné parmi P, Si et Al.

5. Utilisation selon l'une quelconque des revendications 1 à 4, dans laquelle, dans les formules (I), (II), (III), (IV) et (V), M est sélectionné parmi Co, Ti et V.

6. Utilisation selon l'une quelconque des revendications 1 à 5, dans laquelle, dans les formules (I), (II), (III), (IV) et (V), X = P ou Si et M = V.

7. Utilisation selon les revendications 1 à 5, dans laquelle, dans les formules (I), (II), (III), (IV) et (V), X = P ou Si et M = Ti.

8. Utilisation selon les revendications 1 à 5, dans laquelle, dans les formules (I), (II), (III), (IV) et (V), X = P ou Si et M = Co.

9. Utilisation selon les revendications 1 à 5, dans laquelle, dans les formules (I), (II), (III), (IV) et (V), X = Al et M = Co, V ou Ti.

10. Utilisation selon l'une quelconque des revendications précédentes, dans laquelle A comprend au moins un cation sélectionné parmi le groupe composé de cations ammonium quaternaire de formule (VI), cations phosphonium quaternaire de formule (VII) et de cations sulfonium tertiaire de formule (VIII),

$$R^1R^2R^3R^4N^+ \qquad (VI)$$

$$R^1R^2R^3R^4P^+ \qquad (VII)$$

$$R^1R^2R^3S^+ \qquad (VIII)$$

dans laquelle les radicaux $R^1$, $R^2$, $R^3$ et $R^4$ sont indépendamment sélectionnés parmi le groupe composé des hydrocarbures en $C_1$ àCso, des polymères et des composés hétérocycliques, dans lesquelles au moins deux des radicaux $R^1$, $R^2$, $R^3$ et, si présent, $R^4$, sont des hydrocarbures $\geq C_8$ et éventuellement au moins l'un des radicaux

$R^1$, $R^2$, $R^3$ et, si présent, $R^4$, font partie d'un cycle ou forment ensemble un cycle avec l'atome d'azote, de phosphore ou de soufre.

11. Utilisation selon l'une quelconque des revendications précédentes, dans laquelle l'(hétéro)polyoxométalate est de formule (II) et A comprend au moins un cation ammonium quaternaire de formule (VI).

12. Utilisation selon l'une quelconque des revendications 1 à 10, dans laquelle l'(hétéro)polyoxométalate est de formule (II) et A comprend au moins un cation phosphonium quaternaire de formule (VII).

13. Utilisation d'un substrat dans ou sur un appareil domestique comprenant au moins un (hétéro)polyoxométalate tel que défini dans l'une quelconque des revendications précédentes, dans lequel le substrat est un corps de polymère ou un revêtement de polymère comprenant un polymère sélectionné parmi le groupe composé des suivants : polypropylène, polyéthylène, poly(téréphtalate d'éthylène), polyester, polyamide, polyuréthane, polyacrylate, polycarbonate, polystyrène, polyimides, polyméthacrylates, polyoxoalkylènes, poly(oxydes de phénylène), polyvinylesters, polyvinyléthers, chlorure de polyvinylidène, acrylonitrile-butadiène-styrène, polyisoprène naturel et synthétique, polybutadiène, caoutchouc de chloroprène, caoutchouc styrène-butadiène, tétrafluoroéthylène, silicone, résines acrylate, résines polyuréthane, résines de silicone, résines de polyester, résines alkyde, résines époxy, résines phénoliques et résines à base d'urée ou d'amine, ou un mélange de ceux-ci, caoutchouc d'éthylène propylène et caoutchouc d'éthylène propylène diène.

14. Utilisation d'un substrat dans ou sur un appareil domestique selon la revendication 13, dans laquelle le au moins un (hétéro)polyoxométalate est l'(hétéro)polyoxométalate selon la revendication 11.

15. Utilisation d'un substrat dans ou sur un appareil domestique selon la revendication 13, dans laquelle le au moins un (hétéro)polyoxométalate est l'(hétéro)polyoxométalate selon la revendication 12.

16. Utilisation d'un substrat dans ou sur un appareil domestique selon l'une quelconque des revendications 13 à 15, dans laquelle le substrat est un corps de polymère, et dans laquelle le polymère est du polypropylène.

17. Appareil domestique, dans lequel un substrat tel que défini dans l'une quelconque des revendications 13 à 16, est utilisé dans ou sur ledit appareil domestique, et dans lequel le substrat comprend au moins un (hétéro)polyoxométalate tel que défini dans l'une quelconque des revendications 1 à 12.

**Fig. 1a: SEM image of a 10% It-2 coated polypropylene test piece after 1 week (with the 10% It-2 coating on the left, without the 10% It-2 coating on the right).**

**Fig. 1b: SEM image of a 10% It-2 coated polypropylene test piece after 2 weeks (with the 10% It-2 coating on the left, without the 10% It-2 coating on the right).**

**Fig. 2a: SEM image of uncoated polypropylene test piece after 1 week.**

**Fig. 2b: SEM image of uncoated polypropylene test piece after 2 weeks.**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201389539 Y
- WO 2014154432 A1
- EP 1141210 B1
- EP 2765136 A1
- WO 2014122225 A1
- EP 0580078 A1
- WO 9410214 A

**Non-patent literature cited in the description**

- **I.A. WEINSTOCK et al.** Selective Transition-Metal Catalysis of Oxygen Delignification Using Water-Soluble Salts of Polyoxometalate (POM) Anions, Part I. Chemical Principles and Process Concepts. *Holzforschung,* 1998, vol. 52, 304-310